(19) Europäisches Patentamt
European Patent Office
Office européen des brevets

(11) **EP 4 103 931 B1**

(12) **EUROPÄISCHE PATENTSCHRIFT**

(45) Veröffentlichungstag und Bekanntmachung des Hinweises auf die Patenterteilung:
**02.10.2024 Patentblatt 2024/40**

(21) Anmeldenummer: **20821133.4**

(22) Anmeldetag: **27.11.2020**

(51) Internationale Patentklassifikation (IPC):
**G01N 21/64** *(2006.01)* **G01N 21/94** *(2006.01)*
**G01N 33/12** *(2006.01)* **G01N 33/02** *(2006.01)*

(52) Gemeinsame Patentklassifikation (CPC):
**G01N 21/6408; G01N 21/643; G01N 21/94; G01N 33/12;** G01N 2021/646

(86) Internationale Anmeldenummer:
**PCT/EP2020/083813**

(87) Internationale Veröffentlichungsnummer:
**WO 2021/160309 (19.08.2021 Gazette 2021/33)**

(54) **ÜBERPRÜFUNG EINER PROBE HINSICHTLICH VERUNREINIGUNGEN**

INSPECTING A SAMPLE WITH REGARD TO CONTAMINATION

CONTRÔLE D'UN ÉCHANTILLON VIS-À-VIS DE LA CONTAMINATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.02.2020 DE 102020001014**

(43) Veröffentlichungstag der Anmeldung:
**21.12.2022 Patentblatt 2022/51**

(73) Patentinhaber: **Technische Hochschule Rosenheim**
**83024 Rosenheim (DE)**

(72) Erfinder:
• **LANGHALS, Heinz**
**85521 Ottobrunn (DE)**
• **VERSEN, Martin**
**83620 Feldkirchen-Westerham (DE)**
• **WOHLSCHLÄGER, Maximilian**
**83714 Miesbach (DE)**

(74) Vertreter: **Koplin, Moritz**
**Anne-Conway-Straße 1**
**28359 Bremen (DE)**

**Beschreibung**

GEBIET

Die vorliegende Erfindung bezieht sich auf die Überprüfung einer Probe. Insbesondere bezieht sich die vorliegende Erfindung auf die Überprüfung einer Nahrungsmittelprobe hinsichtlich Kunststoff-Verunreinigungen und einer Qualitätsabnahme bedingt durch eine intensive Lichteinstrahlung.

HINTERGRUND

Materialien und Produkte wie bspw. Nahrungsmittel können während ihrer Herstellung oder danach verunreinigt werden. Besagte Verunreinigungen können je nach Art der die Verunreinigung herbeiführenden Substanz gesundheitsschädlich und zugleich mit dem bloßen Auge schwer zu erkennen sein. Neben Verunreinigungen kann die Qualität von Nahrungsmitteln auch durch falsche Lagerung und insbesondere durch intensive Lichteinstrahlung abnehmen.

BOUCHARD ALAIN ET AL, "Optical characterization of Pseudomonas fluorescens on meat surfaces using time-resolved fluorescence", JOURNAL OF BIOMEDICAL OPTICS, Band 11, Nr. 1, 01. Januar 2006 (2006-01-01), Seite 014011, stellt in diesem Zusammenhang ein optisches System vor, welches Bakterien auf Fleischoberflächen anhand der Autofluoreszenz der Bakterien erfassen kann.

Aus der DE 103 15 541 A1 ist ein Verfahren zur Bestimmung des Frischegrades von Lebensmitteln mit wenigstens einer Energiequelle, von der ein Energiestrom auf das zu untersuchende Lebensmittel gerichtet wird, so dass das Lebensmittel zur Autofluoreszenz angeregt wird und Fluoreszenzstrahlung emittiert, sowie mit einem Detektor, der die vom Lebensmittel emittierte Fluoreszenzstrahlung erfasst, wobei die erfasste Fluoreszenzstrahlung in Abhängigkeit ihrer Wellenlänge und/oder Strahlungsintensität im Detektor Detektorsignale hervorruft, die mit Referenzwerten verglichen und unter Zugrundelegung eines Gütekriteriums für eine Aussage über den Frischegrad des Lebensmittels herangezogen werden, bekannt.

Aus der EP 2 251 675 A1 ist ein Verfahren zur Erkennung von tumorbehaftetem Zellgewebe an lebenden entnommenen Zellgewebeproben, insbesondere an Prostatazellgewebe, bekannt.

DARSTELLUNG DER ERFINDUNG

Es ist die Aufgabe der vorliegenden Erfindung Verfahren und Geräte bereitzustellen, die ein Überprüfen von Proben hinsichtlich Verunreinigungen vereinfachen. Ein erfindungsgemäßes Verfahren zur Überprüfung einer Probe hinsichtlich einer Verunreinigung umfasst ein Bestimmen einer Abweichung einer Abklingzeitverteilung einer durch eine elektromagnetische Strahlung angeregten Autofluoreszenz der Probe von einer erwarteten Abklingzeitverteilung, wobei die Verunreinigung Kunststoff, Glas, Metall oder Keramik umfasst oder aus Kunststoff, Glas, Metall oder Keramik besteht.

Das Verfahren kann ferner ein Anzeigen einer Verunreinigung der Probe umfassen, wenn die Abweichung der Verunreinigung zugeordnet ist.

Dabei ist unter dem Begriff "Probe", wie er in der Beschreibung und den Ansprüchen verwendet wird, insbesondere Wasser oder ein organisches Material zu verstehen. Das organische Material kann bspw. ein Nahrungsmittel tierischen oder pflanzlichen Ursprungs sein, wie z. B. Fleisch, Käse, Obst, Gemüse, etc. In diesem Zusammenhang ist unter dem Begriff "Verunreinigung", wie er in der Beschreibung und den Ansprüchen verwendet wird, insbesondere eine (feste oder flüssige) Substanz zu verstehen, die nicht zum Verzehr vorgesehen (oder geeignet) ist, wie bspw. ein Kunststoff und insbesondere Kunststoffpartikel kleiner als 5 mm (Mikroplastik), kleiner als 1 mm, kleiner als 0,1 mm oder kleiner als 0,01 mm. Das organische Material kann ferner eine Stuhl- oder Gewebeprobe sein. Des Weiteren sind unter dem Begriff "Abklingzeitverteilung", wie er in der Beschreibung und den Ansprüchen verwendet wird, insbesondere Daten zu verstehen, aus denen sich eine Abklingzeitstatistik ableiten lässt.

Ferner ist unter dem Begriff "elektromagnetische Strahlung", wie er in der Beschreibung und den Ansprüchen verwendet wird, insbesondere eine elektromagnetische Strahlung eines schmalen Wellenlängenbereichs zu verstehen, wie bspw. Laser-Strahlung. Die elektromagnetische Strahlung kann bspw. eine Wellenlänge im Bereich von 425 nm bis 550 nm aufweisen, wie z. B. 488 nm. Zudem kann die elektromagnetische Strahlung frequenz-, phasen- oder amplitudenmoduliert sein.

Ferner ist unter dem Begriff "Autofluoreszenz", wie er in der Beschreibung und den Ansprüchen verwendet wird, insbesondere eine spontane (wellenlängenverschobene) Emission elektromagnetischer Strahlung durch das Probenmaterial bzw. durch die Verunreinigung zu verstehen, wobei die spontane Emission (verzögert) auf die Anregung des Probenmaterials (durch besagte elektromagnetische Strahlung) folgt.

Das Anzeigen einer Verunreinigung kann bspw. akustisch, haptisch, visuell oder durch ein elektrisches Signal erfolgen. Bspw. kann das elektrische Signal bewirken, dass ein Lebensmittel, das als verunreinigt erkannt wurde, aussortiert und/oder gereinigt oder ggf. entsorgt wird. Zudem kann das elektrische Signal dazu verwendet werden, Lebensmittel je nach Verunreinigungsgrad zu klassifizieren bzw. zu sortieren.

Das Verfahren kann ferner ein Zerlegen der Abklingzeitverteilung der durch die elektromagnetische Strahlung angeregten Autofluoreszenz der Probe in einen oder mehrere einem Material der Probe zugeordnete Anteile und einen oder mehrere Restanteile, umfassen.

Die Abklingzeitverteilung kann bspw. (näherungsweise) in zwei oder mehr (sich ggf. teilweise überlappende) Verteilungen zerlegbar sein. Die Verteilungen können zu lokalen Maxima der Abklingzeitverteilung symmetrisch sein. Zum Zerlegen können somit in einem ersten Schritt die lokalen Maxima der Abklingzeitverteilung bestimmt werden und in einem zweiten Schritt die Abklingzeitverteilung in symmetrische Verteilungen (bspw. Gaußverteilungen) zerlegt werden, deren Scheitelpunkte mit den lokalen Maxima (im Wesentlichen) übereinstimmen.

Das Verfahren kann ferner ein Abgleichen des einen oder der mehreren dem Material der Probe zugeordneten Anteile mit einer charakteristischen Abklingzeitverteilung eines Nahrungsmittels umfassen.

Bspw. kann bestimmt werden, ob eine der (Gauß-)verteilungen einer charakteristischen Abklingzeitverteilung der Probe zugeordnet werden kann.

Die charakteristische Abklingzeitverteilung kann aus einer Referenzmessung oder aus einer Vielzahl an Referenzmessungen (bspw. durch Mittelung) abgeleitet oder künstlich (als idealisierte Abklingzeitverteilung) erzeugt werden.

Das Verfahren kann ferner ein Abgleichen des einen oder der mehreren Restanteile mit einer Abklingzeitverteilung der Verunreinigung umfassen.

Bspw. kann eine durchschnittliche Abklingzeit einer einem Restanteil zugeordneten (Gauß-)verteilung oder eine durchschnittliche Abklingzeit des Restanteils mit durchschnittlichen Abklingzeiten bekannter möglicher Verunreinigungen verglichen und bei Übereinstimmung einer dieser Verunreinigungen zugeordnet werden.

Die Verunreinigung kann bspw. einen Kunststoff umfassen oder aus einem Kunststoff bestehen, z.B. Polyamid, Polyethylen, Polyvinylchlorid, Polyethylenterephthalat, Polystyrol, Polypropylen, oder Acrylnitril-Butadien-Styrol, der eine charakteristische Abklingzeitverteilung aufweist.

Ein Restanteil, der keiner bekannten möglichen Verunreinigung zugeordnet werden kann, bspw. weil zwischen dem Restanteil und den (Gauß-)verteilungen, die bekannten Verunreinigungen zugeordnet sind, keine oder nur eine (zu) geringe Übereinstimmung besteht, kann dahingehend analysiert werden, ob er ausgeprägte lokale Maxima aufweist, die ggf. auf eine unbekannte Verunreinigung hinweisen oder, ob er keine ausgeprägten Maxima aufweist und eher als Messrauschen zu interpretieren ist.

Ferner kann eine Verunreinigung einen Bereich des Probenmaterials abschatten und dadurch eine Änderung des Fluoreszenzspektrums bewirken.

Das Verfahren kann ferner ein Vergleichen von Abklingzeitverteilungen der durch die elektromagnetische Strahlung angeregten Autofluoreszenz von zwei oder mehr unterschiedlichen Probenvolumina oder Proben umfassen. Eine Verunreinigung kann hierbei bspw. dann angezeigt werden, wenn eine Abweichung der zwei oder mehr Abklingzeitverteilungen voneinander außerhalb eines Toleranzbereichs liegt.

Als Maß der Abweichung kann bspw. eine Funktion des Abstandes zwischen den Scheitelpunkten der Verteilungen, ein Grad der Überlappung der Verteilungen, ein Unterschied in der Breite der Verteilungen oder eine (gewichtete) Kombination zweier oder aller dieser Faktoren verwendet werden.

Bspw. kann eine Abklingzeitverteilung einer Probe oder eine aus mehreren Abklingzeitverteilungen unterschiedlicher Proben abgeleitete (bspw. gemittelte) Abklingzeitverteilung als Referenz verwendet werden. Ferner kann eine Referenz durch eine gleitende Mittelung über mehrere gemessene Abklingzeitverteilungen, die auf keine Verunreinigungen schließen lassen, erzeugt werden.

Die mittels gleitender Mittelung über mehrere gemessene Abklingzeitverteilungen ermittelte Referenz kann ferner zur Erfassung von Trends verwendet werden.

Durch einen fortlaufenden Vergleich kann zudem die Homogenität der Proben überprüft werden.

Das Verfahren kann ferner ein Bestimmen einer Verschiebung der Abklingzeitverteilung relativ zu einer Referenz und ein Anzeigen einer Qualitätsverschlechterung umfassen, wenn die Abklingzeitverteilung außerhalb eines Toleranzbereichs um die Referenz liegt.

Dabei ist unter der Formulierung "Verschiebung der Abklingzeitverteilung", wie sie in der Beschreibung und den Ansprüchen verwendet wird, insbesondere eine Reduktion einer (mittleren) Abklingzeit zu verstehen. Eine solche Reduktion der (mittleren) Abklingzeit kann bspw. durch anhaltende Lichteinstrahlung bewirkt werden. Als Maß für die Verschiebung kann bspw. eine Funktion des Abstandes zwischen (einander entsprechenden) lokalen Maxima der Verteilungen und/oder ein Grad der Überlappung der Verteilungen dienen.

Das erfindungsgemäße Gerät zur Überprüfung einer Probe umfasst einen Emitter, eingerichtet zur Aussendung eines frequenz-, phasen- oder amplitudenmodulierten Lichts und einen Detektor, eingerichtet zur Aufnahme einer durch die elektromagnetische Strahlung angeregten Autofluoreszenz der Probe, wobei das Gerät eingerichtet ist, ein Ergebnis der Überprüfung auf Basis einer Abklingzeitverteilung der Autofluoreszenz der Probe zu bestimmen. Das Gerät ist ferner eingerichtet, eine Verunreinigung der Probe anzuzeigen, wenn die Abklingzeitverteilung der Autofluoreszenz der Probe von einer erwarteten Abklingzeitverteilung abweicht, wobei die Verunreinigung Kunststoff, Glas, Metall oder Keramik umfasst oder aus Kunststoff, Glas, Metall oder Keramik besteht.

Das Gerät kann des Weiteren eingerichtet sein, einen Probenumriss zu erfassen.

Das Erfassen des Probenumrisses kann dazu dienen, Störeinflüsse an den Probenrändern zu reduzieren. Bspw. kann das Gerät nur Messwerte verwenden, die sich auf Messstellen innerhalb des Probenumrisses und/oder in einem bestimmten Abstand zum Probenumriss befinden.

Das Gerät kann ferner eingerichtet sein, die Verunreinigung relativ zum Probenumriss zu lokalisieren.

Ferner können aus dem Probenumriss die Größe und Form der Verunreinigung abgeleitet werden.

Das Gerät kann des Weiteren eingerichtet sein, eine Qualität der Probe zu beanstanden, wenn eine Abklingzeit der Autofluoreszenz geringere Werte aufweist, als erwartet.

Das Gerät kann ferner ein Messer umfassen und zum Zerteilen eines Lebensmittels eingerichtet sein.

Bspw. kann das Gerät zum Schneiden von Fleisch oder Fleischprodukten (insbesondere Wurst) eingerichtet sein, so dass evtl. beim Schneiden in die Wurst gelangende Verpackungsteile erfasst werden können.

Zudem versteht es sich, dass die im Zusammenhang mit dem Gerät beschriebenen Merkmale auch Merkmale des Verfahrens sein können, in dem das Gerät verwendet werden kann und umgekehrt.

KURZE BESCHREIBUNG DER ZEICHNUNGEN

Die Erfindung wird nachfolgend in der detaillierten Beschreibung anhand von Ausführungsbeispielen erläutert, wobei auf Zeichnungen Bezug genommen wird, in denen:

Fig. 1 eine schematische Ansicht eines Geräts zur Überprüfung einer Probe hinsichtlich Verunreinigungen/Qualität zeigt;

Fig. 1a und Fig. 1b das in Fig. 1 gezeigte Gerät mit Einrichtungen zum Anzeigen eines Ergebnisses der Überprüfung illustrieren;

Fig. 2 eine mögliche Modifikation der in Fig. 1 schematisch illustrierten Anordnung zeigt;

Fig. 2a und Fig. 2b illustrieren, wie damit umgegangen werden kann, wenn in der in Fig. 2 gezeigten Anordnung eine Verunreinigung einer Probe angezeigt wird;

Fig. 3 eine weitere mögliche Modifikation der in Fig. 1 schematisch illustrierten Anordnung zeigt;

Fig. 3a und Fig. 3b die Funktion einer Einrichtung zum Anzeigen eines Ergebnisses einer Überprüfung, welche durch die in Fig. 3 gezeigte Anordnung durchgeführt wurde, illustrieren;

Fig. 4 ein Vorgehen zum Bestimmen der Abklingzeit einer durch eine elektromagnetische Strahlung angeregten Autofluoreszenz der Probe schematisch illustriert;

Fig. 5 eine Unterteilung einer Probe in mehrere (Mess-)Segmente schematisch illustriert;

Fig. 6 eine beispielhafte Abklingzeitverteilung einer nicht verunreinigten Probe zeigt;

Fig. 6a eine beispielhafte Abklingzeitverteilung einer verunreinigten Probe zeigt;

Fig. 6b eine weitere beispielhafte Abklingzeitverteilung einer verunreinigten Probe zeigt;

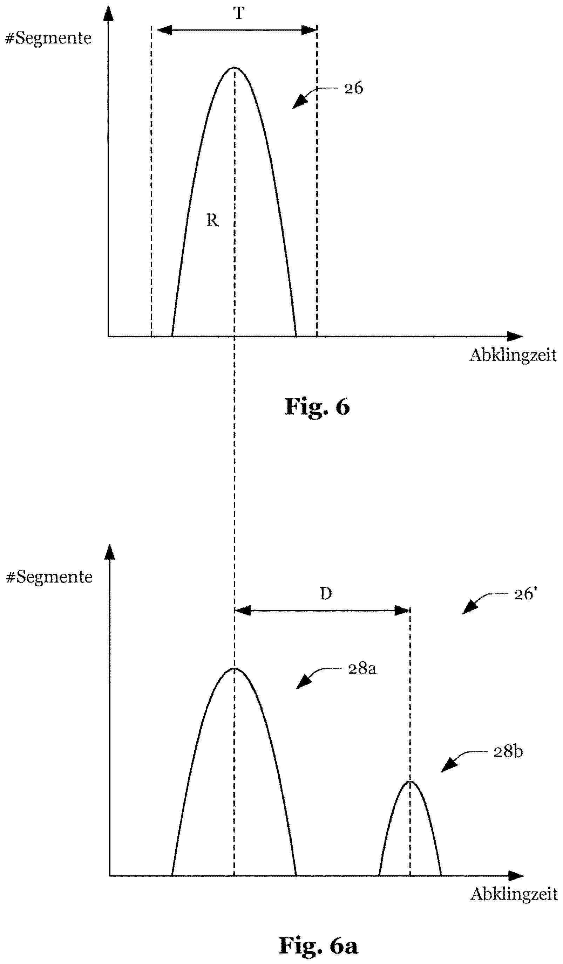

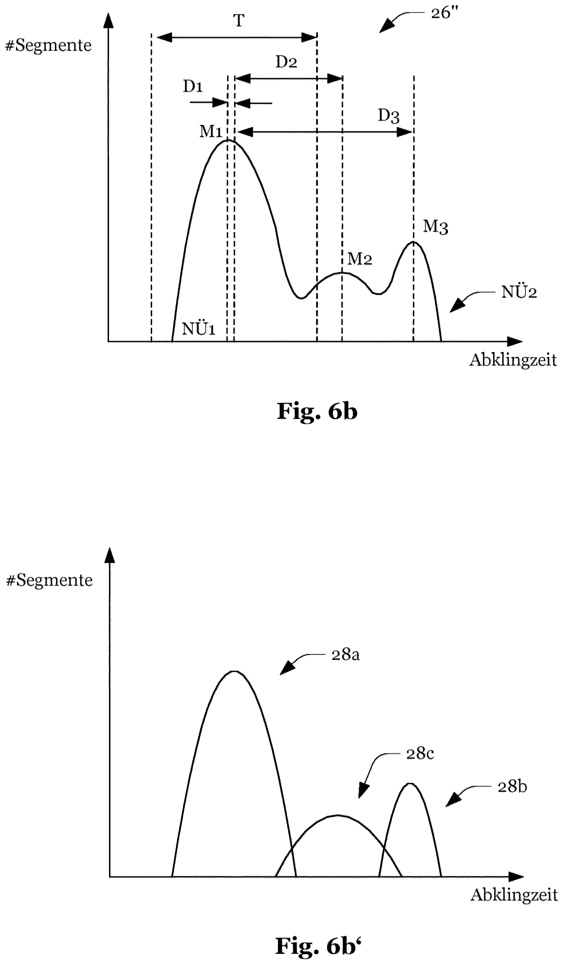

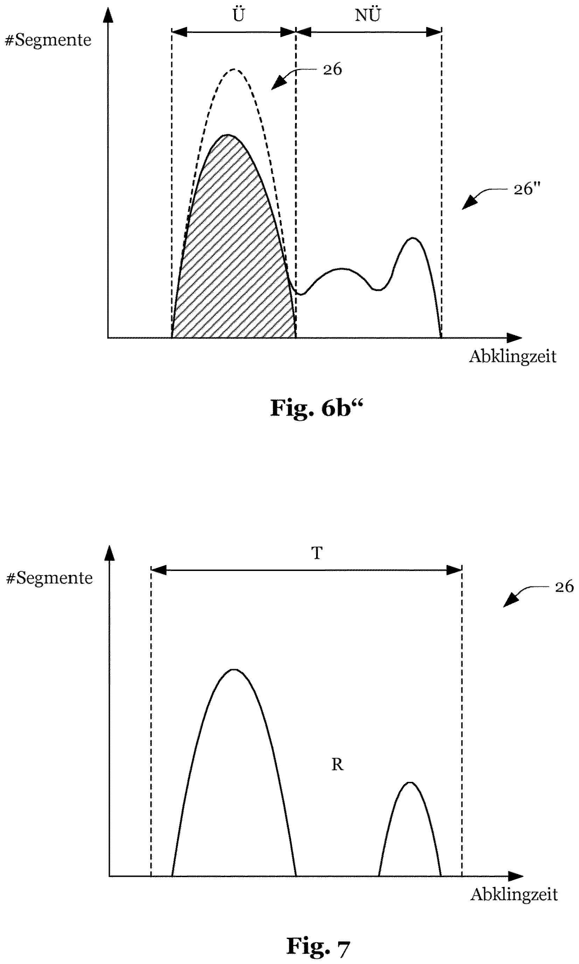

Fig. 6b' eine Zerteilung der in Fig. 6b gezeigten Abklingzeitverteilung in einen dem Probenmaterial zugeordneten Anteile und zwei Restanteile schematisch illustriert;

Fig. 6b" eine Bestimmung eines Maßes für die Abweichung der in Fig. 6b gezeigten Abklingzeitverteilung von einer Referenz schematisch illustriert;

Fig. 7 eine weitere beispielhafte Abklingzeitverteilung einer verunreinigten Probe zeigt;

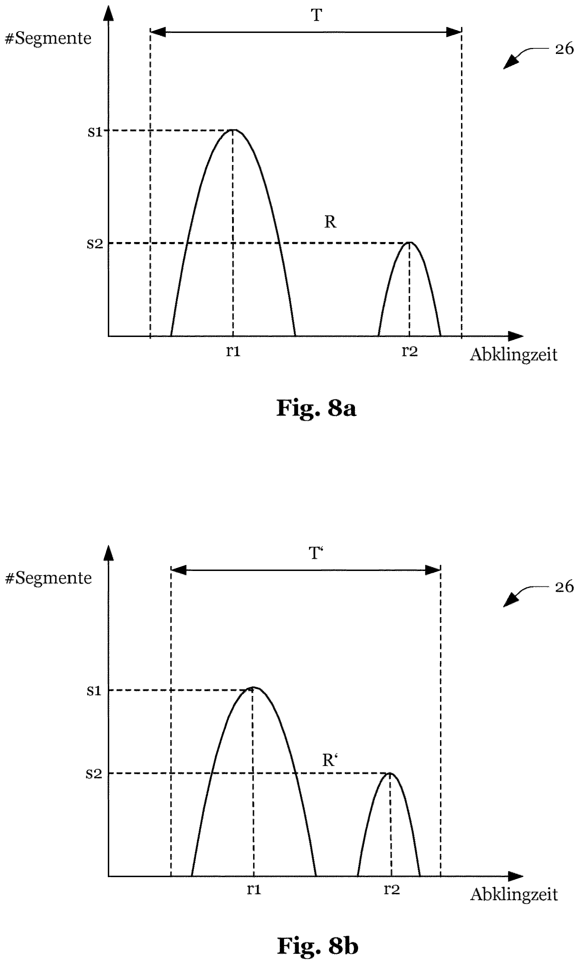

Fig. 8a und Fig. 8b die Verwendung einer gleitenden Referenz schematisch illustrieren;

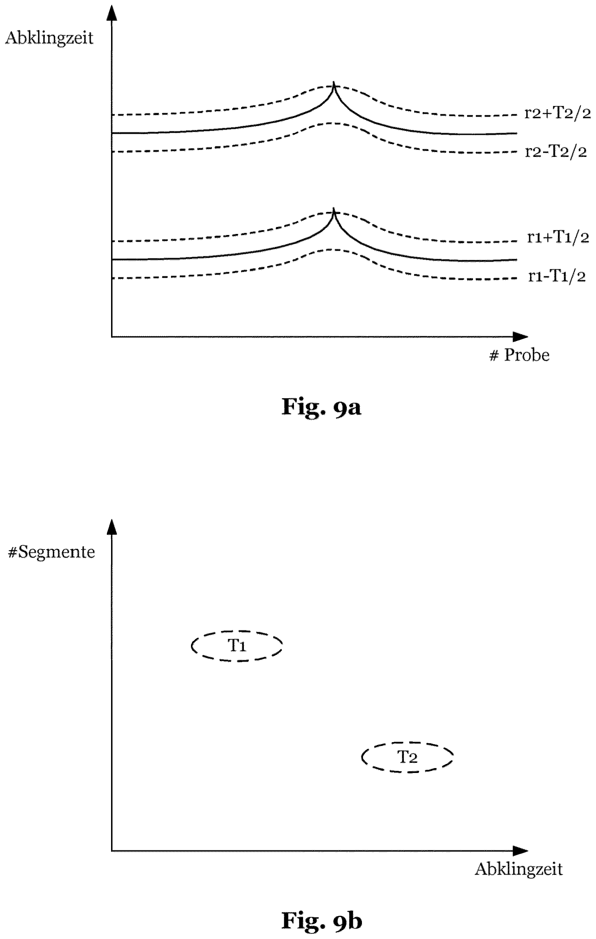

Fig. 9a und Fig. 9b die Verwendung einer gleitenden und/oder mehrdimensionalen Referenz illustrieren;

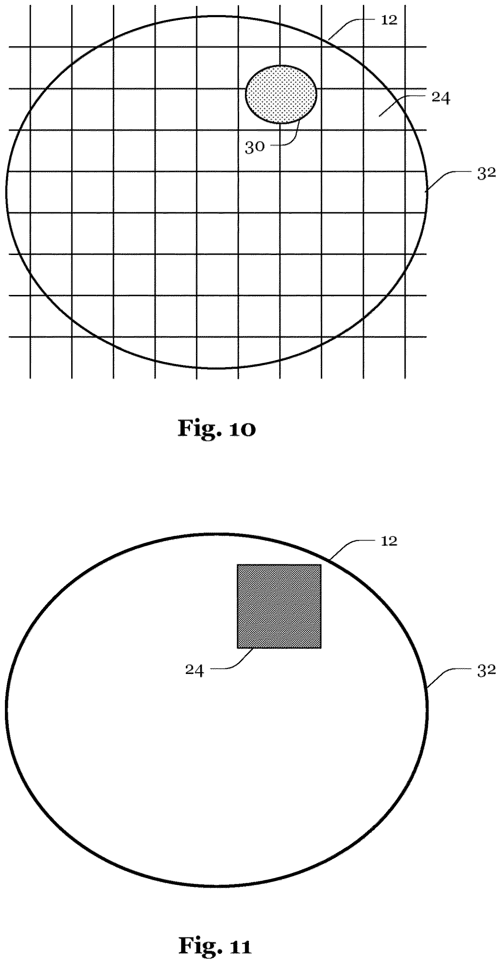

Fig. 10 das Lokalisieren einer Verunreinigung schematisch illustriert;

Fig. 11 das Anzeigen der in Fig. 10 lokalisierten Verunreinigung schematisch illustriert;

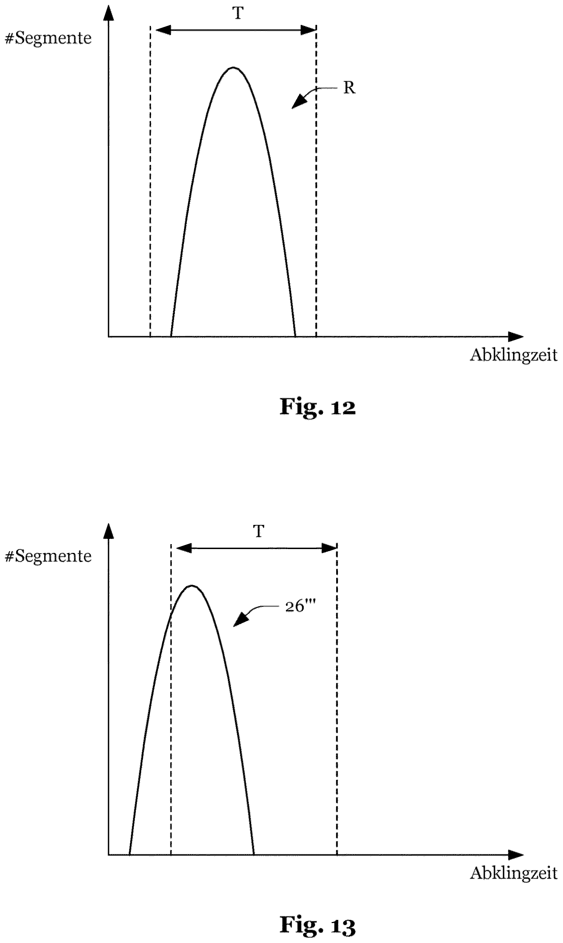

Fig. 12 und Fig. 13 eine Verschiebung der Abklingzeitverteilung schematisch illustrieren;

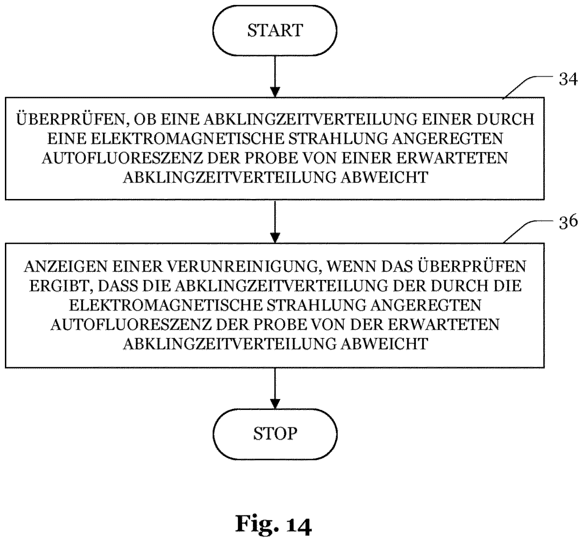

Fig. 14 ein Flussdiagramm eines beispielhaften Verfahrens zeigt;

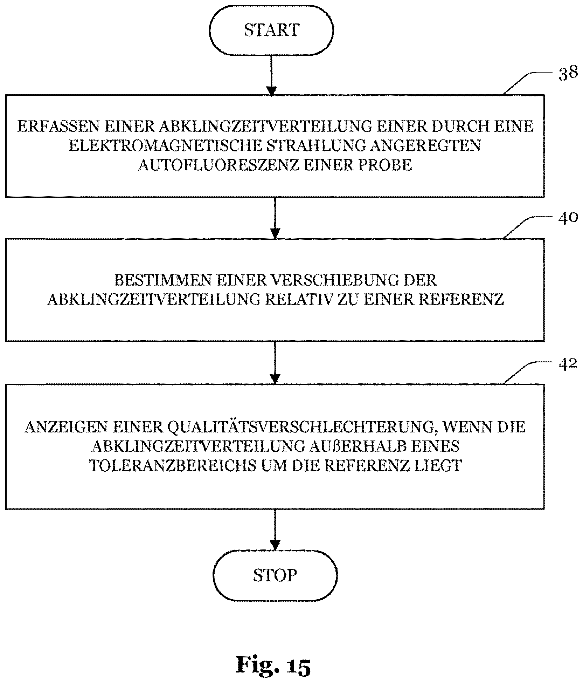

Fig. 15 ein Flussdiagramm eines weiteren beispielhaften Verfahrens zeigt;

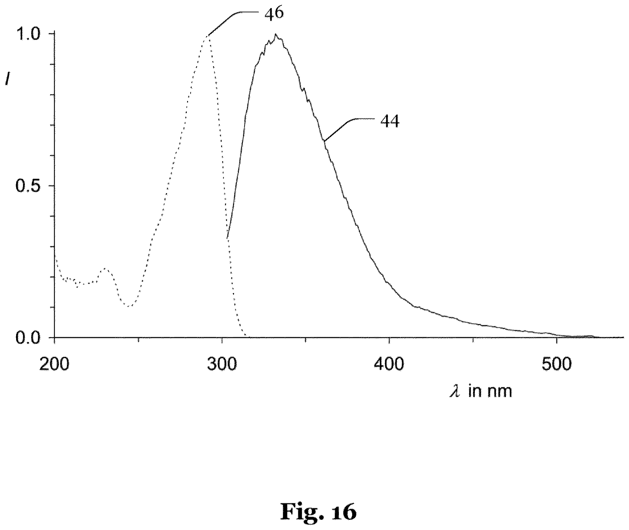

Fig. 16 ein Fluoreszenzanregungs- und Fluoreszenzspektrum einer Salami aus Schweine- und Rindfleisch zeigt;

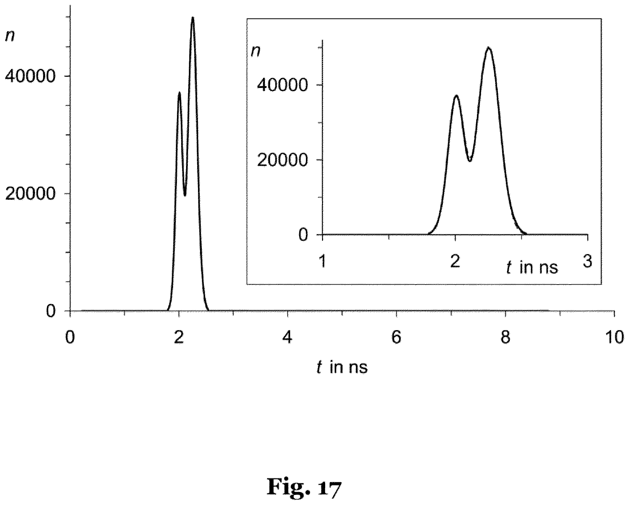

Fig. 17 das Fluoreszenzabklingverhalten von Putensalami nach Anregung mittels Laserlicht mit einer Wellenlänge von 488 nm illustriert;

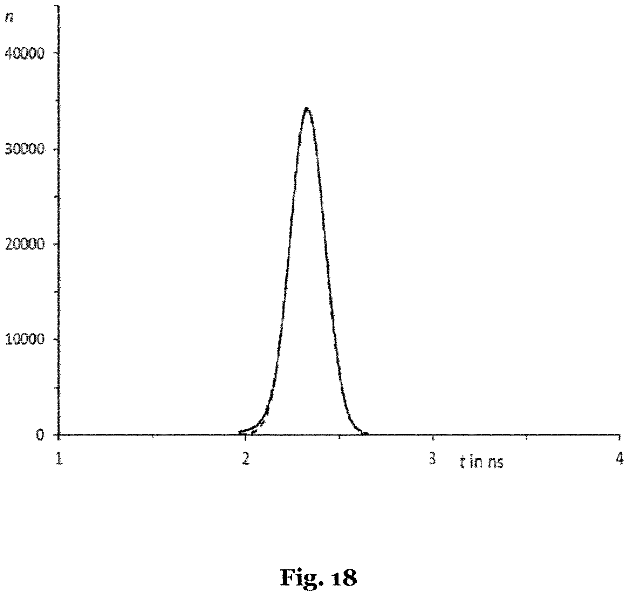

Fig. 18 das Fluoreszenzabklingverhalten von Rindersalami nach Anregung mittels Laserlicht mit einer Wellenlänge von 488 nm illustriert;

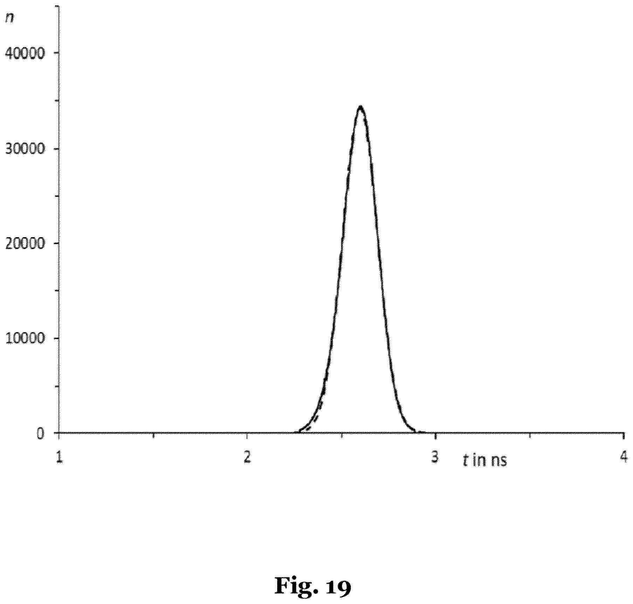

Fig. 19 das Fluoreszenzabklingverhalten von Schweinesalami nach Anregung mittels Laserlicht mit einer Wellenlänge von 488 nm illustriert;

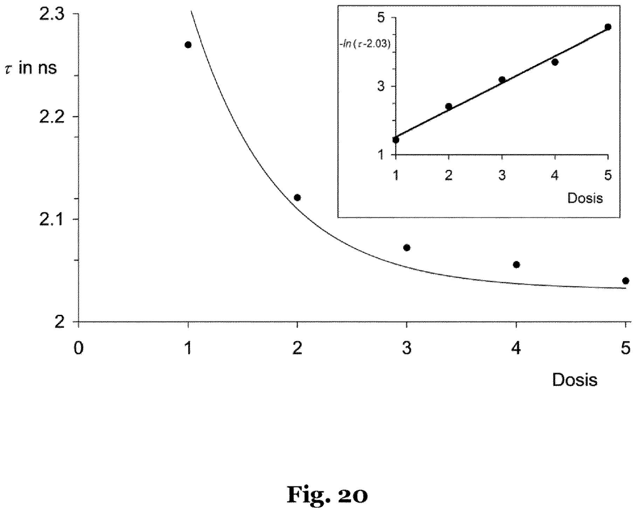

Fig. 20 die Änderung der Fluoreszenzabkling-Zeitkonstante der Putensalami als Funktion der Bestrahlungs-Dosis illustriert;

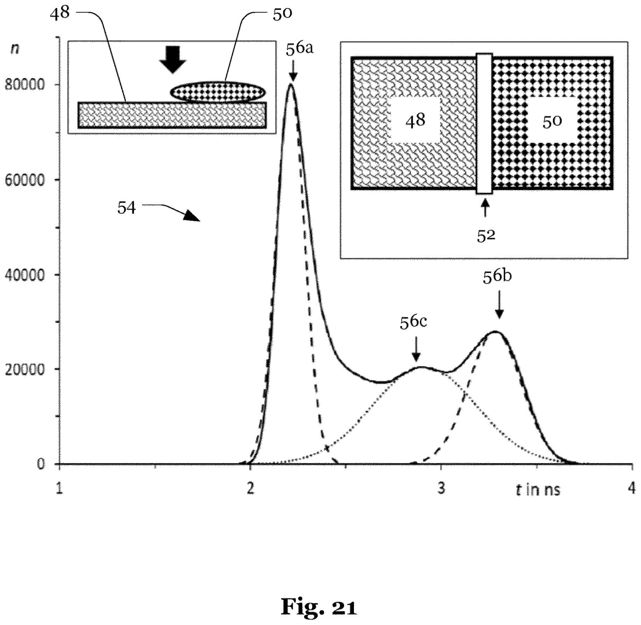

Fig. 21 das Fluoreszenzabklingverhalten von Putensalami, welche mit Polyamid verunreinigt wurde, in einem Randbereich der Verunreinigung illustriert;

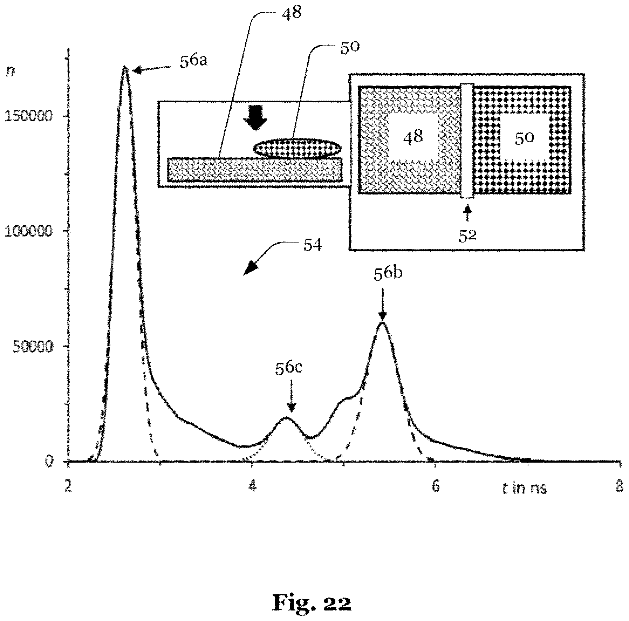

Fig. 22 das Fluoreszenzabklingverhalten von Putensalami, welche mit PVC verunreinigt wurde, in einem Randbereich der Verunreinigung illustriert;

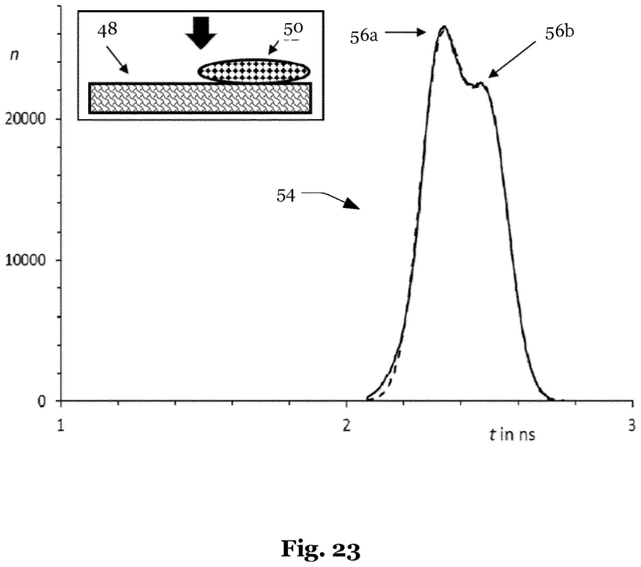

Fig. 23 das Fluoreszenzabklingverhalten von Putensalami, welche mit PET verunreinigt wurde, in einem Randbereich der Verunreinigung illustriert;

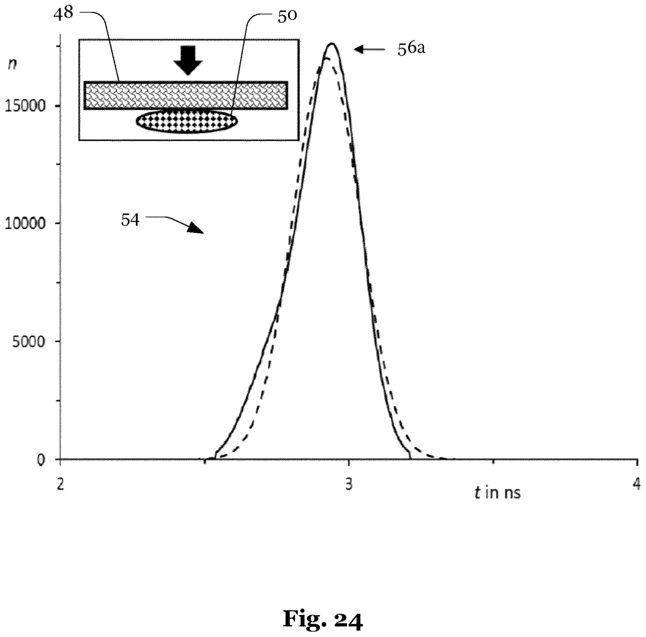

Fig. 24 das Fluoreszenzabklingverhalten von Putensalami, welche mit Polyamid auf einer der Einstrahlung gegenüberliegenden Seite verunreinigt wurde, illustriert;

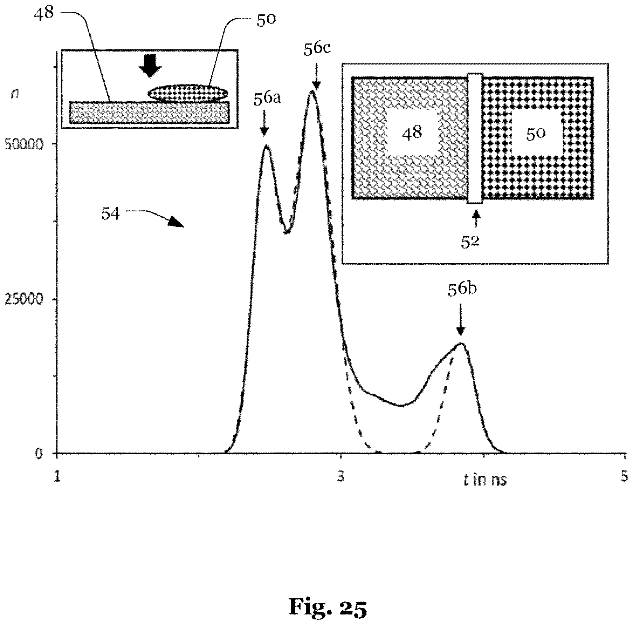

Fig. 25 das Fluoreszenzabklingverhalten von Rindersalami, welche mit einem Kunststoff verunreinigt wurde, in einem Randbereich der Verunreinigung illustriert;

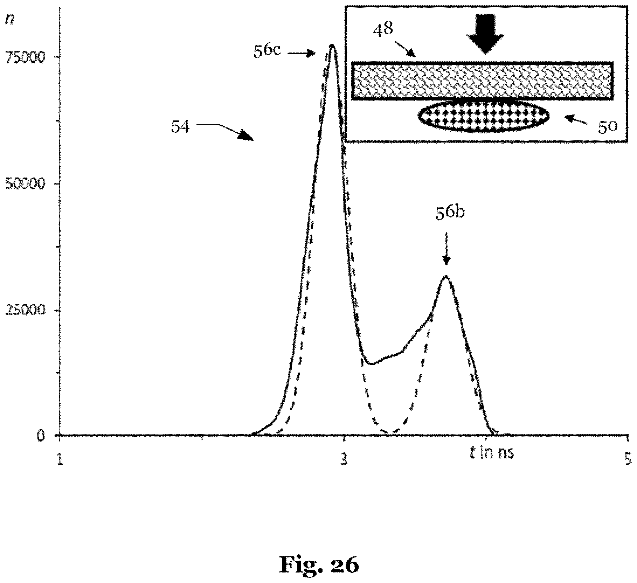

Fig. 26 das Fluoreszenzabklingverhalten von Rindersalami, welche mit Kunststoff auf einer der Einstrahlung gegenüberliegenden Seite verunreinigt wurde, illustriert;

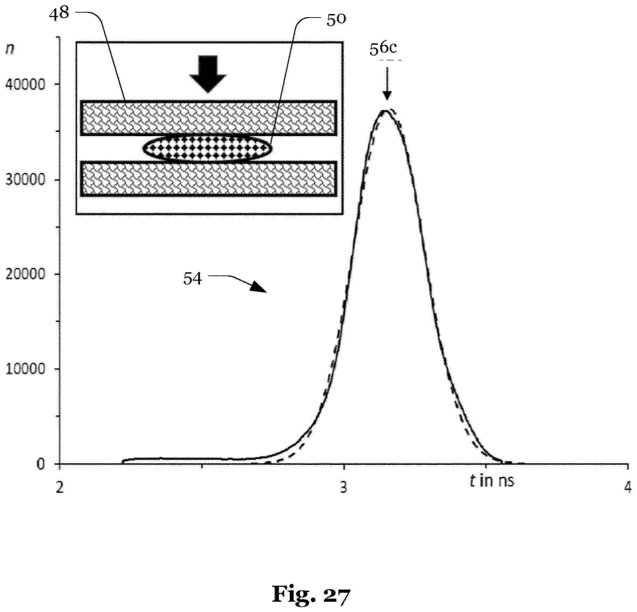

Fig. 27 das Fluoreszenzabklingverhalten von zwei Rindersalamischeiben, zwischen denen ein Kunststoffstück angeordnet ist, illustriert;

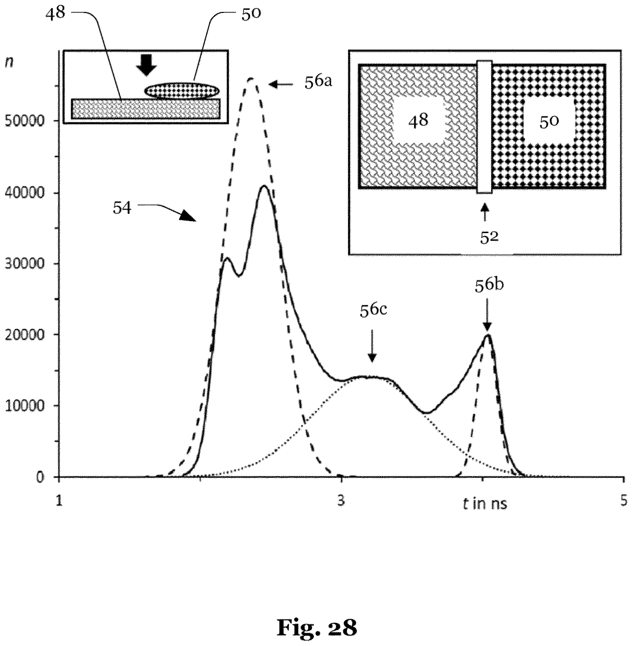

Fig. 28 das Fluoreszenzabklingverhalten von Schweinesalami, welche mit einem Kunststoff verunreinigt wurde, in einem Randbereich der Verunreinigung illustriert;

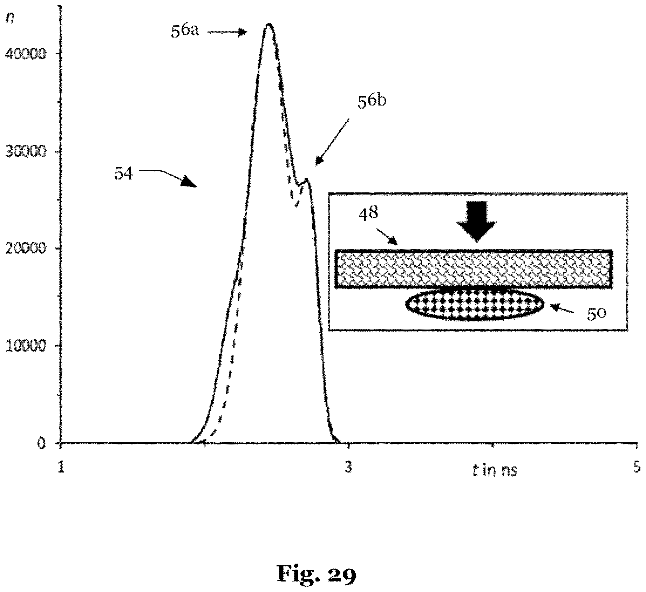

Fig. 29 das Fluoreszenzabklingverhalten von Schweinesalami, welche mit Kunststoff auf einer der Einstrahlung gegenüberliegenden Seite verunreinigt wurde, illustriert;

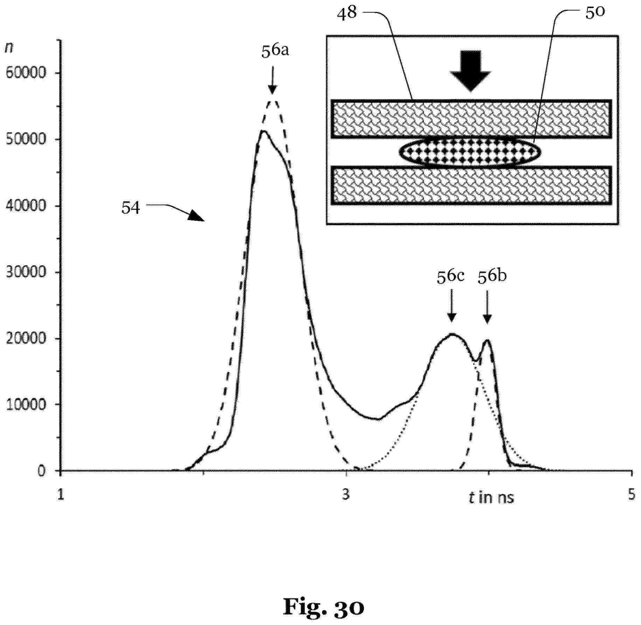

Fig. 30 das Fluoreszenzabklingverhalten von zwei Schweinesalamischeiben, zwischen denen ein Kunststoffstück angeordnet ist, illustriert;

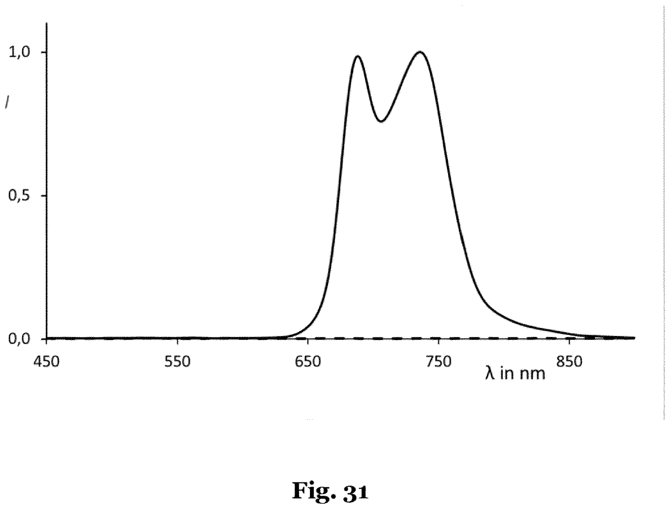

Fig. 31 das Fluoreszenzspektrum eines Salatblatts illustriert;

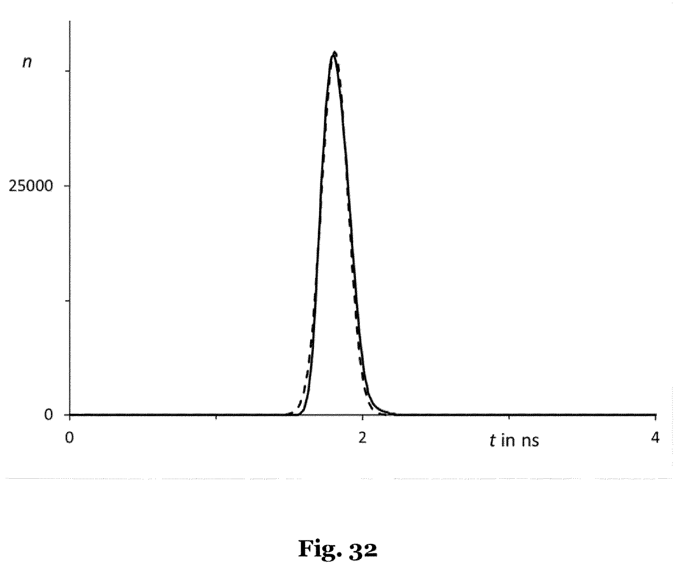

Fig. 32 das Fluoreszenzabklingverhalten eines Salatblattes illustriert;

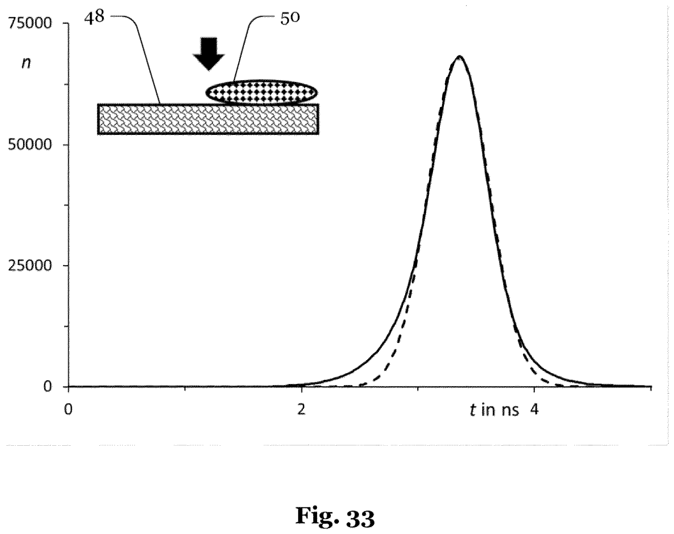

Fig. 33 das Fluoreszenzabklingverhalten eines Salatblattes illustriert, welches auf der Oberseite mit rotem HDPE Kunststoff verunreinigt wurde;

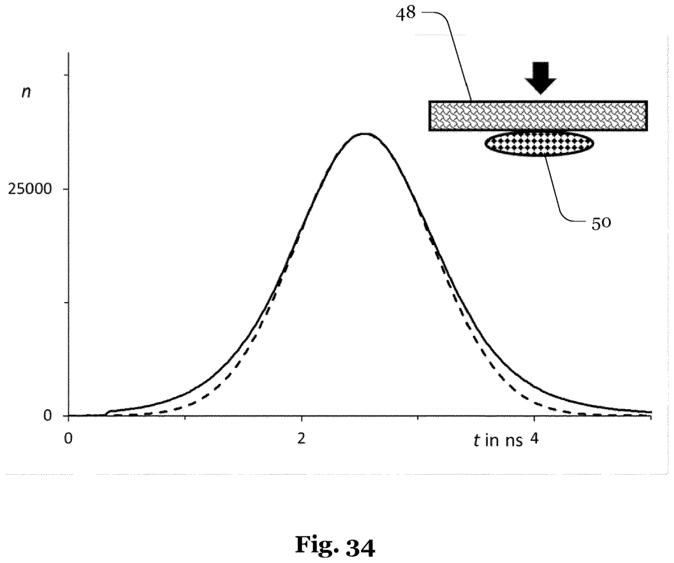

Fig. 34 das Fluoreszenzabklingverhalten eines Salatblattes illustriert, welches auf der Unterseite mit rotem HDPE Kunststoff verunreinigt wurde;

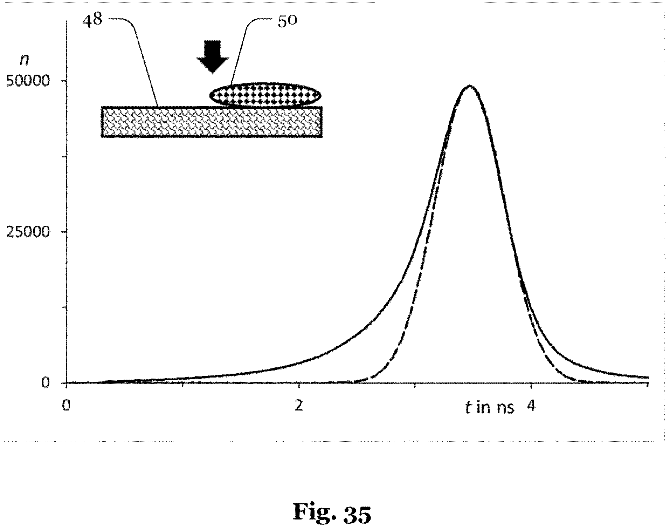

Fig. 35 das Fluoreszenzabklingverhalten eines Salatbreis illustriert, welcher auf der Oberseite mit rotem HDPE Kunststoff verunreinigt wurde;

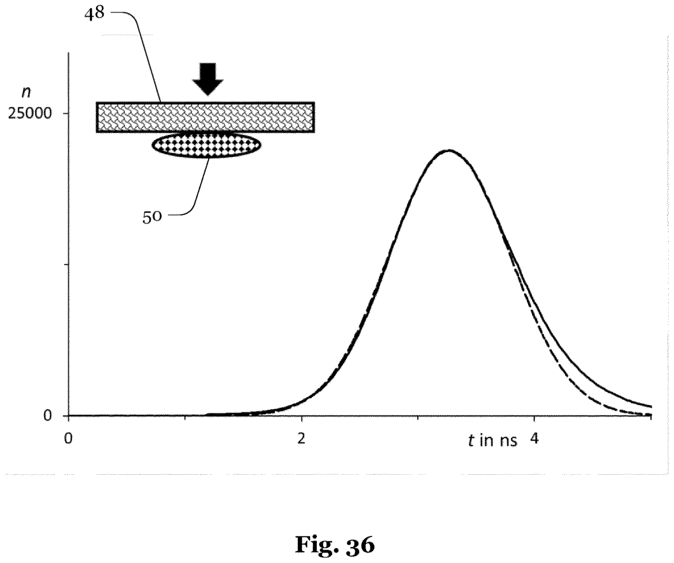

Fig. 36 das Fluoreszenzabklingverhalten eines Salatbreis illustriert, welcher unter einer dünnen Schicht mit dem roten HDPE Kunststoff verunreinigt wurde;

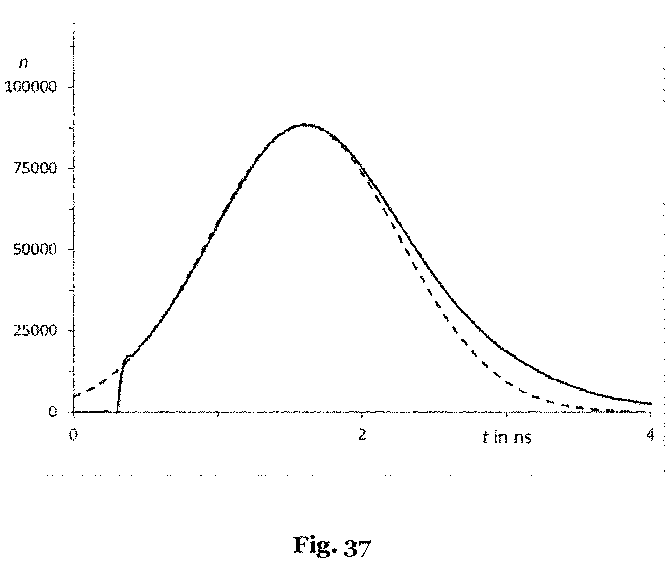

Fig. 37 das Fluoreszenzabklingverhalten von Schinken illustriert;

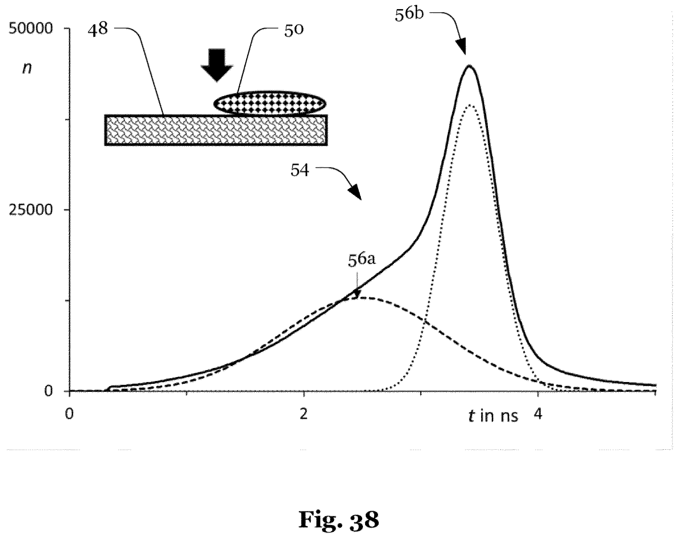

Fig. 38 das Fluoreszenzabklingverhalten von Schinken illustriert, welcher mit rotem HDPE Kunststoff auf der Oberfläche verunreinigt wurde;

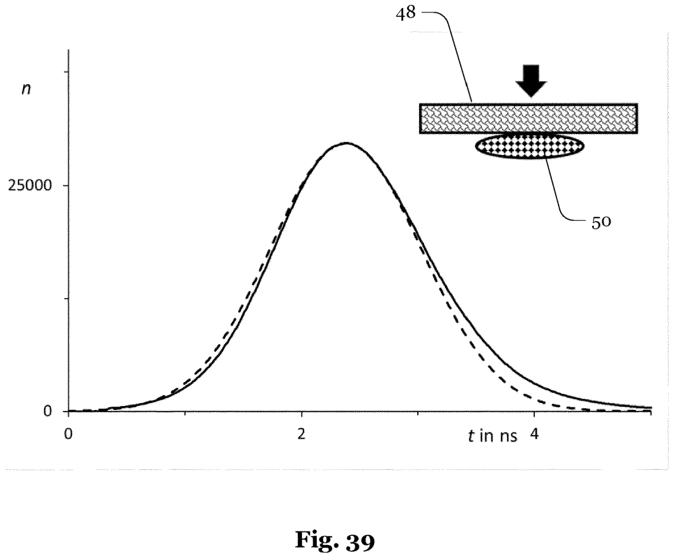

Fig. 39 das Fluoreszenzabklingverhalten von Schinken illustriert, welcher mit rotem HDPE Kunststoff an der Unterseite verunreinigt wurde;

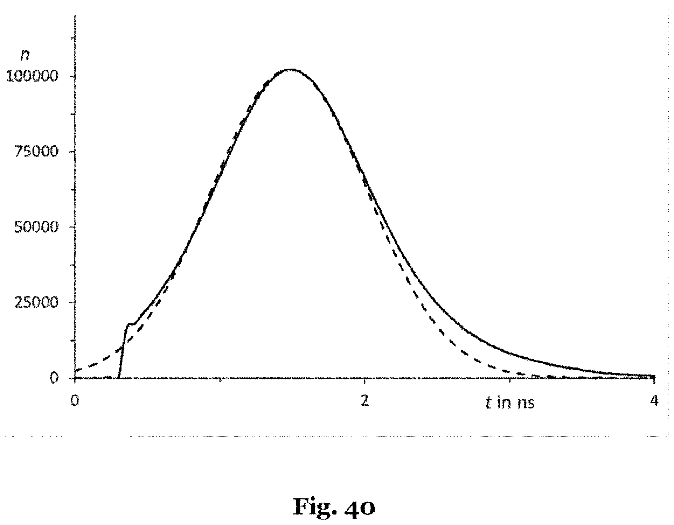

Fig. 40 das Fluoreszenzabklingverhalten von Fisch illustriert;

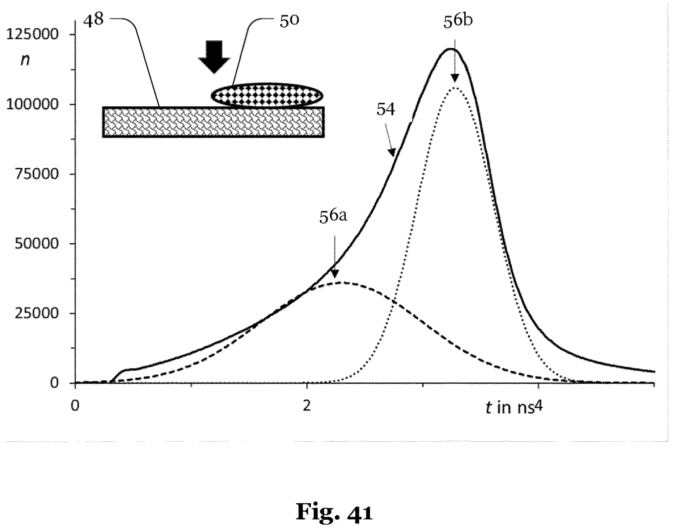

Fig. 41 das Fluoreszenzabklingverhalten von Fisch illustriert, welcher an der Oberfläche mit HDPE Kunststoff verunreinigt wurde;

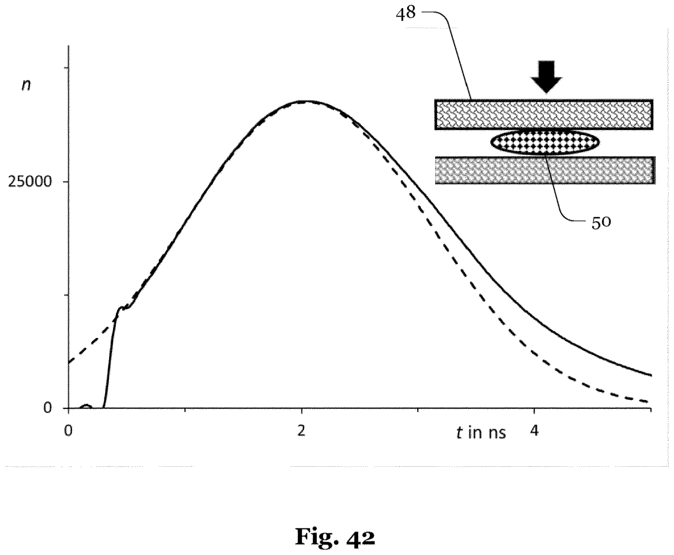

Fig. 42 das Fluoreszenzabklingverhalten von Fisch illustriert, welcher im Inneren mit rotem HDPE Kunststoff verunreinigt wurde;

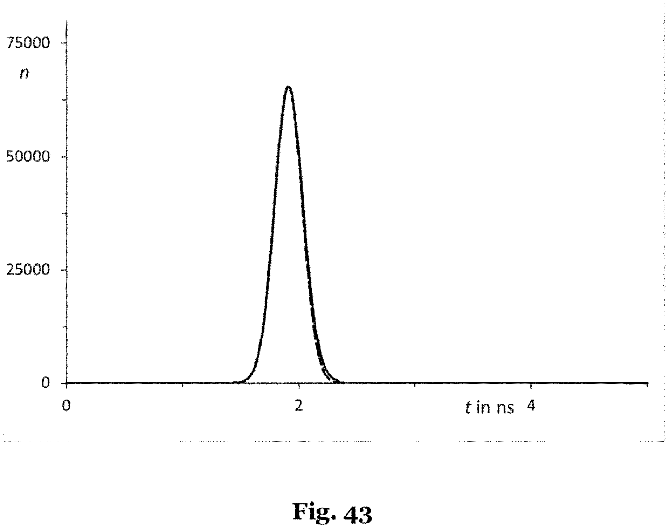

Fig. 43 das Fluoreszenzabklingverhalten von Honig illustriert;

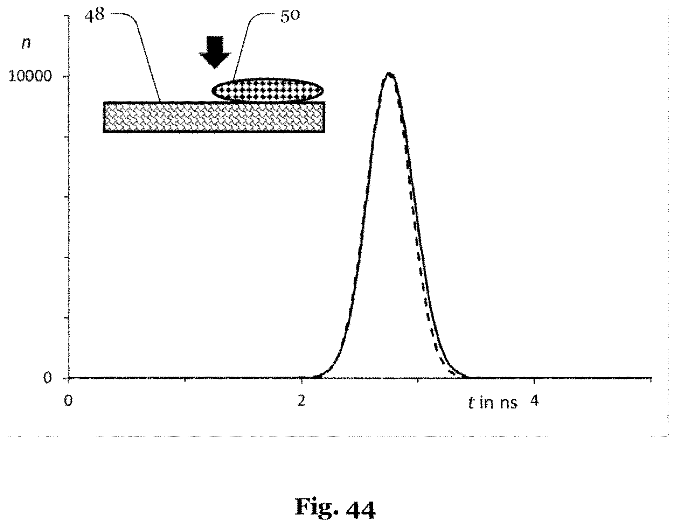

Fig. 44 das Fluoreszenzabklingverhalten von Honig illustriert, welcher eine oberflächennahe Verunreinigung mit rotem HDPE aufweist;

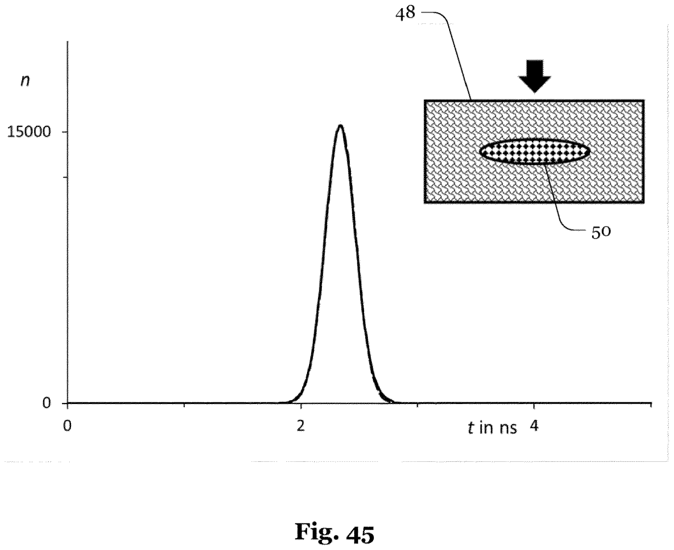

Fig. 45 das Fluoreszenzabklingverhalten von Honig illustriert, welcher eine Verunreinigung mit rotem HDPE 3 mm unter der Oberfläche aufweist;

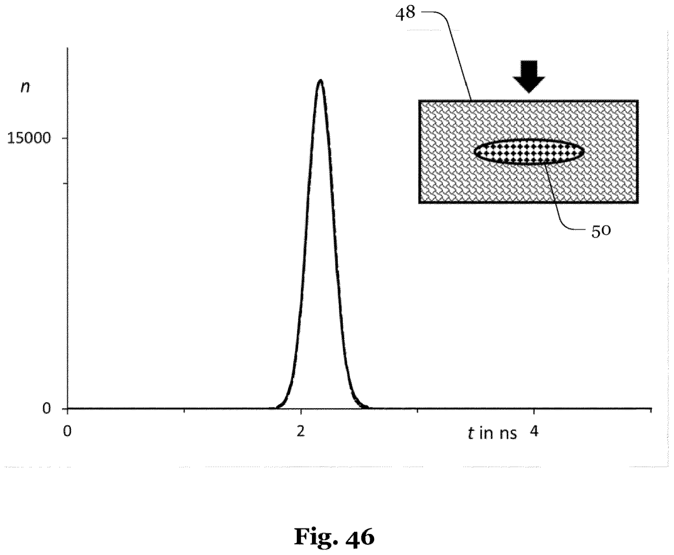

Fig. 46 das Fluoreszenzabklingverhalten von Honig illustriert, welcher eine Verunreinigung mit rotem HDPE 5 mm unter der Oberfläche aufweist; und

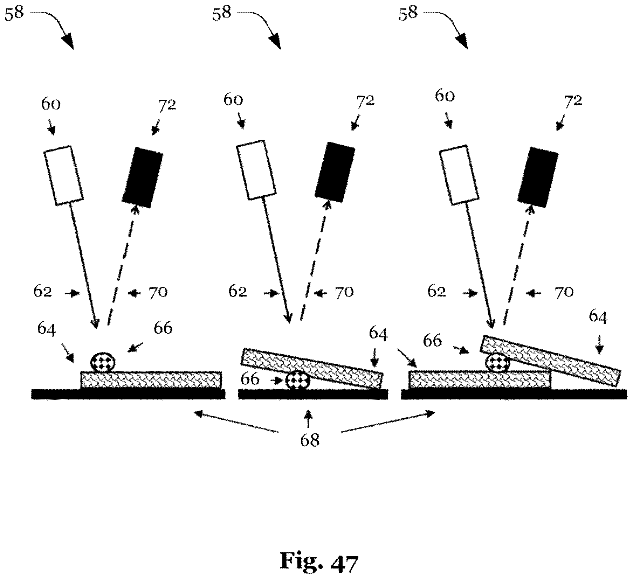

Fig. 47 Systeme zur Kontrolle und Detektion von (Kunststoff-) Verunreinigungen in Lebensmitteln in der Lebensmittelverarbeitung illustriert.

Dabei sind in den Zeichnungen gleiche oder funktional ähnliche Elemente durch gleiche Bezugszeichen gekennzeichnet.

## WEGE ZUR AUSFÜHRUNG DER ERFINDUNG

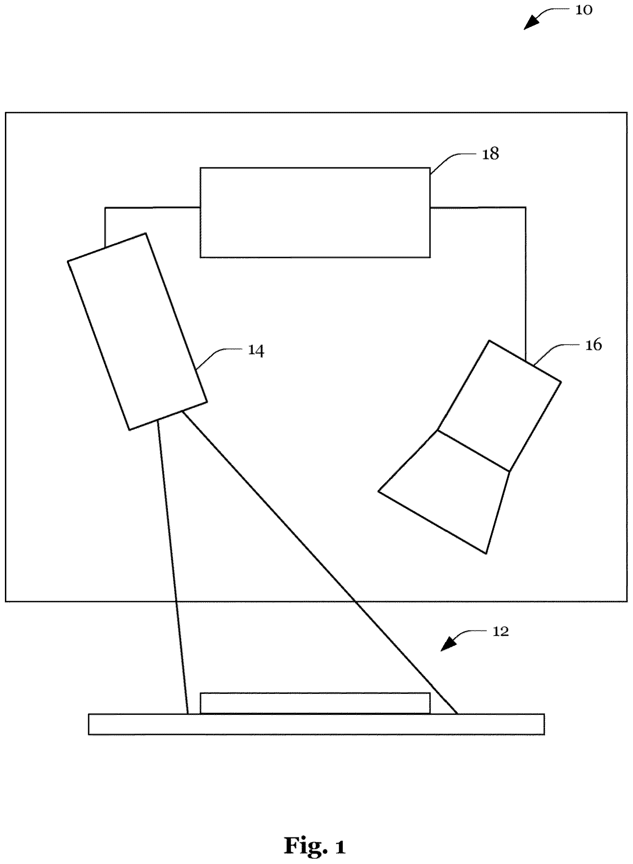

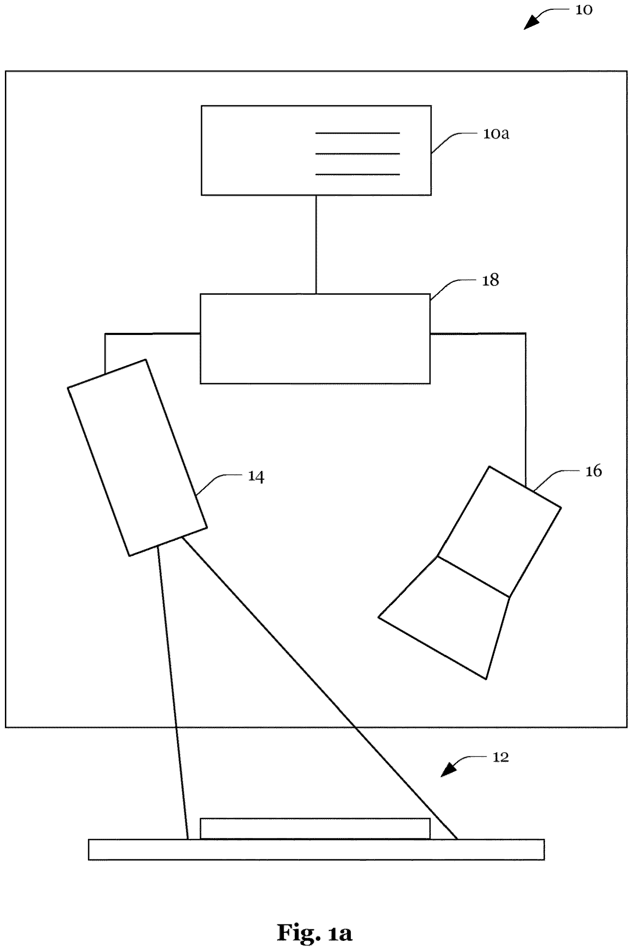

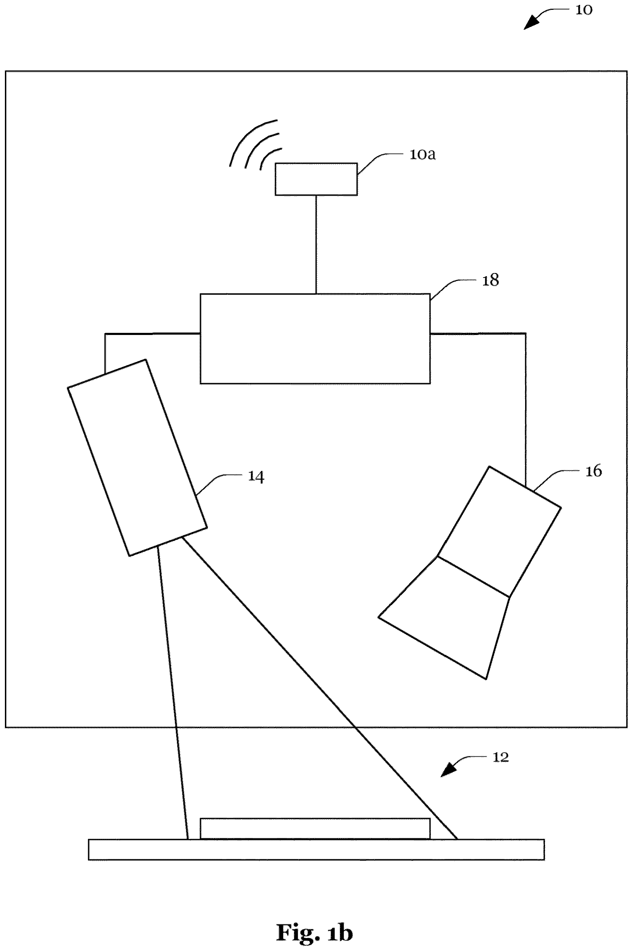

Fig. 1 zeigt Gerät 10, welches der Überprüfung von Probe 12 dient. Gerät 10 umfasst Emitter 14 und Detektor 16. Emitter 14 ist zur Aussendung einer amplitudenmodulierten elektromagnetischen Strahlung eingerichtet. Detektor 16 ist zur Aufnahme einer durch die elektromagnetische Strahlung angeregten Autofluoreszenz (von Probe 12) eingerichtet. Gerät 10 umfasst ferner Rechnereinheit 18, welche eingerichtet ist, ein Ergebnis der Überprüfung auf Basis einer Abklingzeitverteilung der Autofluoreszenz zu bestimmen. Wie in Fig. 1a und Fig. 1b illustriert, kann das Ergebnis einem Benutzer von Gerät 10 auf Anzeigeeinrichtung 10a visuell oder akustisch angezeigt werden.

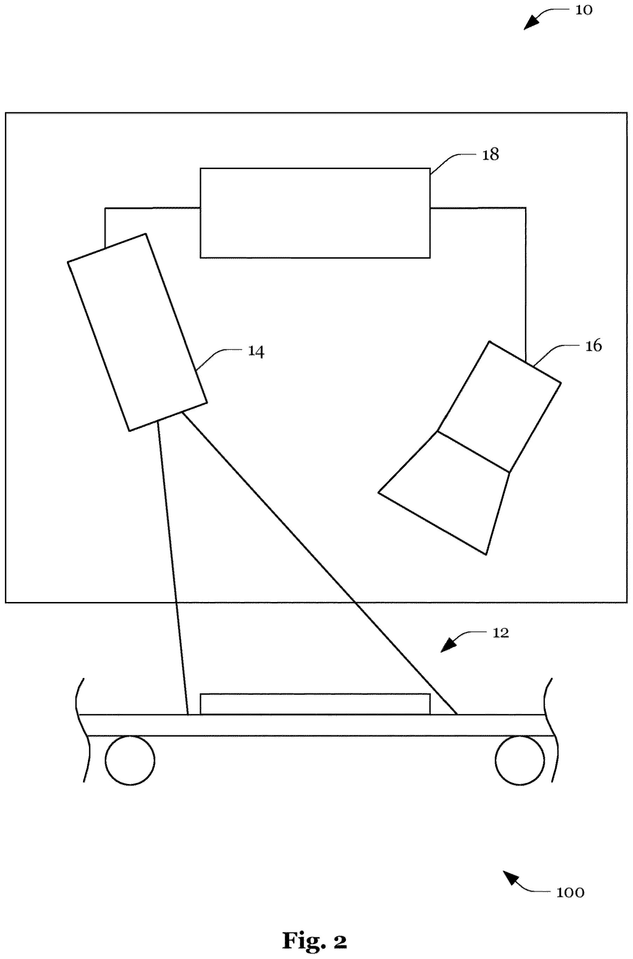

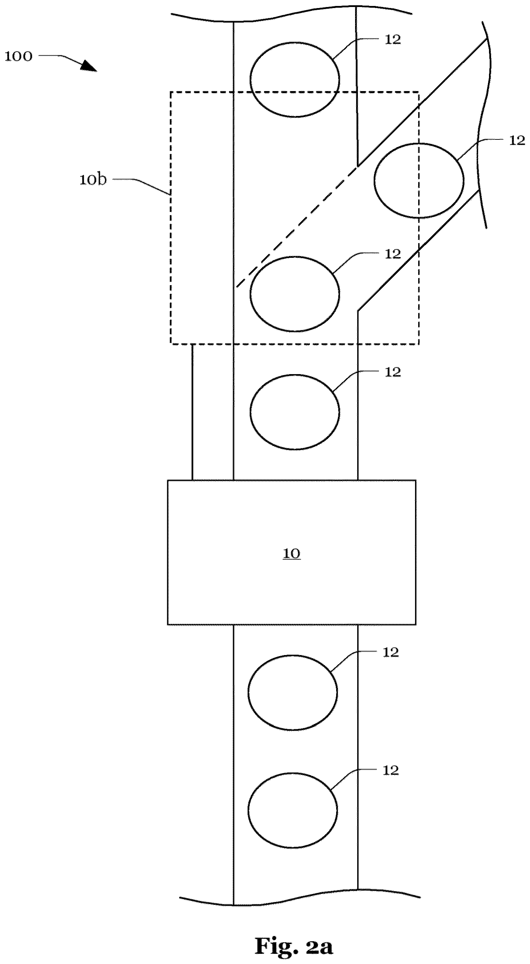

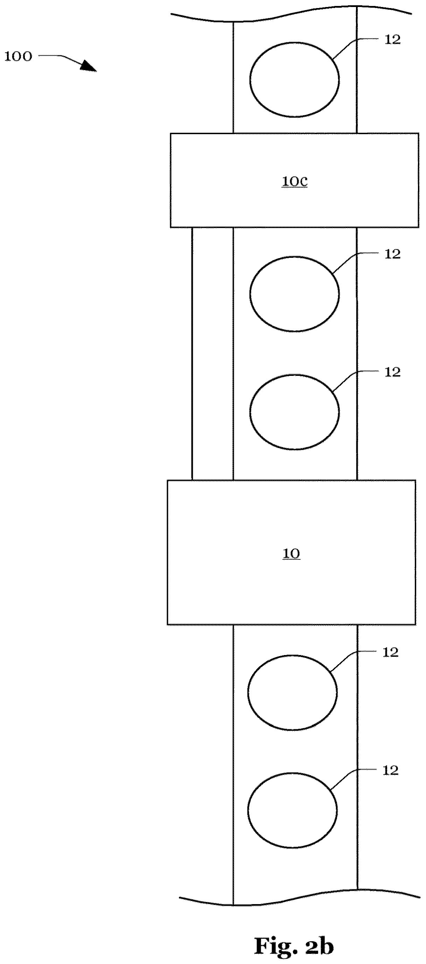

Fig. 2 zeigt eine mögliche Modifikation der in Fig. 1 gezeigten Anordnung, in der Proben 12 mittels Fördersystem 100 an Gerät 10 vorbeibewegt werden. Rechnereinheit 18 kann dabei die Fördergeschwindigkeit von Fördersystem 100 überwachen und den Überwachungsprozess mit der Fördergeschwindigkeit synchronisieren. Ferner kann Rechnereinheit 18 eine Verunreinigung einer Probe anzeigen, woraufhin Fördersystem 100 die Probe, wie in Fig. 2a illustriert, mittels Einrichtung 10b aussortieren oder wie in Fig. 2b illustriert, mittels Einrichtung 10c von der Verunreinigung befreien kann.

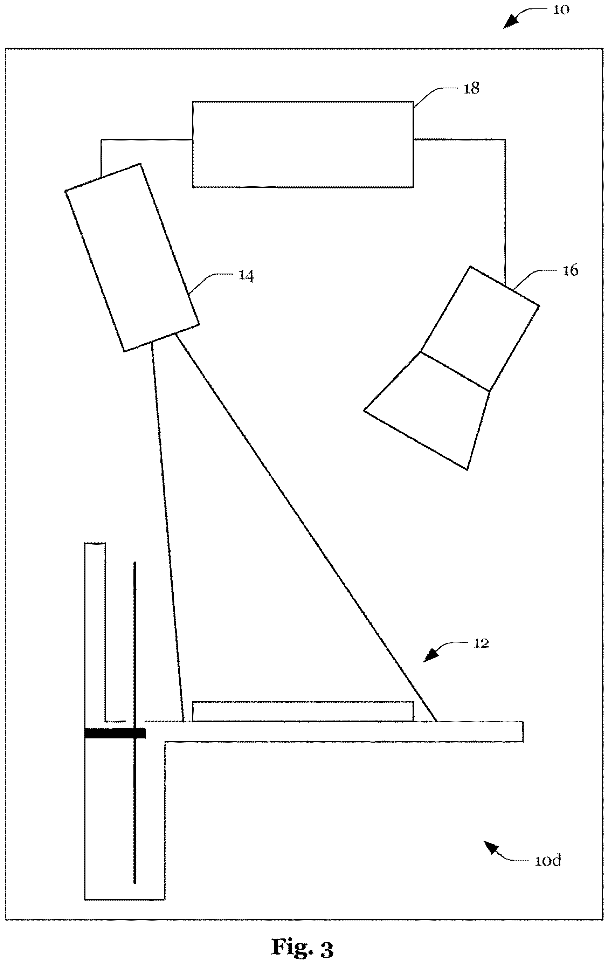

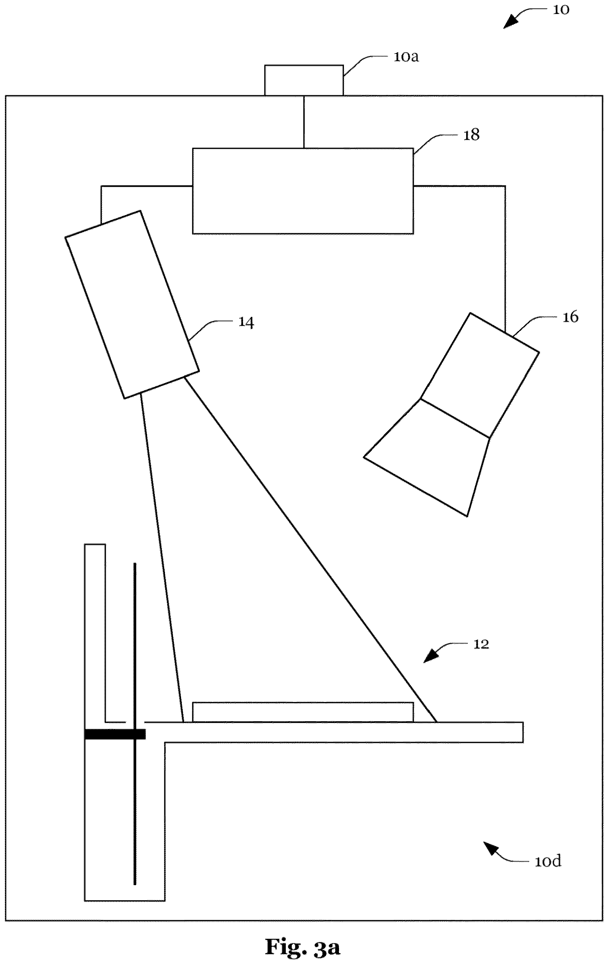

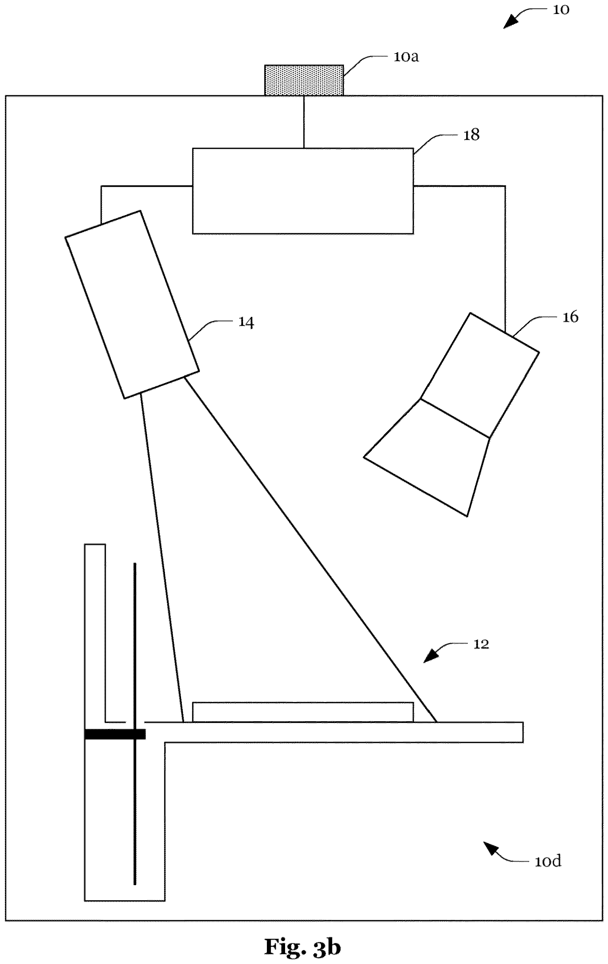

Fig. 3 zeigt eine weitere mögliche Modifikation der in Fig. 1 gezeigten Anordnung. In der in Fig. 3 gezeigten Anordnung umfasst Gerät 10 Schneidemaschine 10d. Schneidemaschine 10d weist ein Messer auf, das zum Zerteilen von Lebensmitteln vorgesehen ist. Schneidemaschine 10d kann bspw. eine Wurstschneidemaschine sein. Gerät 10 kann eingerichtet sein, zu überwachen, ob die Wurst beim Schneiden bspw. mit Kunststoff verunreinigt wird. Zu diesem Zweck kann Detektor 16 so ausgerichtet sein, dass die geschnittenen Wurstscheiben (Proben 12) nach dem Schneiden im Erfassungsbereich von Detektor 16 zu liegen kommen.

Wie in Fig. 3a und Fig. 3b illustriert, kann das Ergebnis einem Benutzer von Gerät 10 mittels Anzeigeeinrichtung 10a (visuell) angezeigt werden. Bspw. kann Gerät 10 ein Signal ausgeben, wenn eine verunreinigte Probe erfasst wird. Z. B. kann, wie in Fig. 3a und 3b illustriert, Anzeigeeinrichtung 10a von einem ersten Zustand (bspw. einer grünen Farbe) in einen zweiten Zustand wechseln (bspw. einer roten Farbe), wenn eine verunreinigte Probe 12 erfasst wird. Ebenso kann Anzeigeeinrichtung 10a ein erstes akustisches oder haptisches Signal ausgeben, wenn eine nicht verunreinigte Probe erfasst wird und ein zweites akustisches oder haptisches Signal, wenn eine verunreinigte Probe erfasst wird. Gerät 10 kann bspw. direkt in einem Verkaufsbereich verwendet werden und sowohl einem Bediener als auch einem Kunden dazu dienen eine Verunreinigung zu erkennen und geeignete Maßnahmen zu treffen.

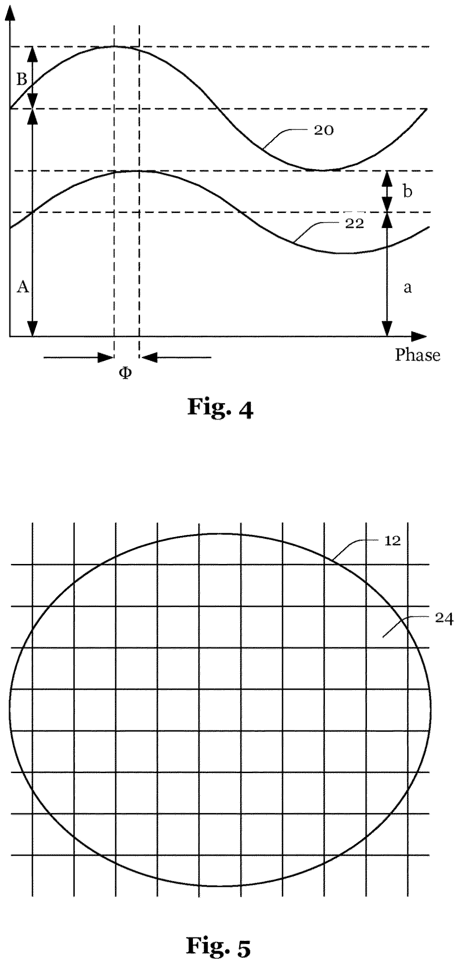

Fig. 4 illustriert eine sinusförmige Amplitudenmodulation 20 der elektromagnetischen Strahlung, welche um Φ phasenverschobene Modulation 22 der Autofluoreszenz bewirkt. Ausgehend von Amplitudenmodulationen 20 und 22 kann

$$\tau_P = \frac{\tan(\Phi)}{\omega}$$

als Maß für die Abklingdauer verwendet werden, wobei $\omega$ die Kreisfrequenz von Amplitudenmodulation 20 angibt.

Wie in Fig. 5 gezeigt, kann Detektor 16 die Oberfläche von Probe 12 in (gleich große) (Mess-)Segmente 24 unterteilen. Detektor 16 kann $\tau_P$ für jedes Segment 24 (einmal oder mehrmals) individuell bestimmen. Aus der Gesamtheit aller $\tau_P$ kann, wie in Fig. 6 illustriert, Abklingzeitverteilung 26 bestimmt werden.

Fig. 6a illustriert eine Veränderung von Abklingzeitverteilung 26, die sich durch eine Verunreinigung ergeben kann. Die in Fig. 6a gezeigte veränderte Abklingzeitverteilung 26' unterscheidet sich von der Abklingzeitverteilung 26 dadurch, dass sie sich (im Wesentlichen) in zwei, nicht-überlappende (Gauß-)verteilungen 28a und 28b zerlegen lässt. Während (Gauß-)verteilung 28a mit Abklingzeitverteilung 26 hinsichtlich der Abklingzeit, an der die Maxima von Verteilung 26 und (Gauß-)verteilung 28a zu finden sind, (im Wesentlichen) übereinstimmt, ist (Gauß-)verteilung 28a im Vergleich zu Abklingzeitverteilung 26 in Richtung höherer Abklingzeiten (um Abstand D) verschoben. In diesem Fall kann Abstand D (oder eine Funktion des Abstands D) als Maß für die Wahrscheinlichkeit des Vorliegens einer Verunreinigung dienen. Weitere Auswertestrategien umfassen das Festlegen eines Toleranzbereiches T und das Anzeigen einer Verunreinigung, falls eine Funktion der außerhalb des Toleranzbereiches T liegenden Segmente von Abklingzeitverteilung 26' einen Schwellenwert über- (oder unterschreitet, das Berechnen einer (spektrumsoder flächenmäßigen) Überlappung von Abklingzeitverteilung 26 und Abklingzeitverteilung 26', etc.

Fig. 6b illustriert eine weitere Veränderung von Abklingzeitverteilung 26, die sich durch eine Verunreinigung ergeben kann. Die in Fig. 6b gezeigte veränderte Abklingzeitverteilung 26" unterscheidet sich von Abklingzeitverteilung 26 dadurch, dass sie, wie in Fig. 6b' illustriert, in drei, sich überlappende (Gauß-)verteilungen 28a, 28b und 28c zerlegbar ist. Dabei ist (Gauß-)verteilung 28a einem nicht verunreinigten Teil von Probe 12 und (Gauß-)verteilung 28b einem verunreinigten Teil von Probe 12 zuordenbar. Ferner ist Gaußverteilung 28c, die mit (Gauß-)verteilung 28a und (Gauß-)verteilung 28b überlappt, einem Übergang zwischen dem nicht verunreinigten Teil und dem verunreinigten Teil von Probe 12 zuordenbar. Die Zerlegung kann (in Fällen geringer Komplexität) bspw. dadurch erfolgen, dass (ggf. nach einer Glättung) lokale Maxima (Scheitelpunkte) M1, M2 und M3 bestimmt werden und ausgehend von den äußeren Maxima M1 und M3 symmetrische Verteilungen an die sich nichtüberlappenden Abschnitte NÜ1 und NÜ2 (oder ggf. an die sich möglichst wenig überlappenden Abschnitte) angepasst werden.

Ferner können, wie in Fig. 6b illustriert, Abstände D1, D2 und/oder D3 (oder eine Funktion derselbigen) zwischen dem Maximum von Abklingzeitverteilung 26 und den lokalen Maxima von Abklingzeitverteilung 26" verwendet werden, um eine Aussage über die Wahrscheinlichkeit des Vorliegens einer Verunreinigung zu treffen. Ebenso kann ein Toleranzbereiche T festgelegt werden und eine Verunreinigung angezeigt werden, falls eine Funktion der außerhalb des Toleranzbereiches T liegenden Segmente von Abklingzeitverteilung 26" einen Schwellenwert über- (oder unterschreitet. Ferner kann, wie in Fig. 6b" illustriert, eine (spektrums- oder flächenmäßige) Überlappung von Abklingzeitverteilung 26 und Abklingzeitverteilung 26" bestimmt werden und bei zu geringer Überlappung auf das Vorhandensein einer Verunreinigung geschlossen werden. Eine zu geringe Überlappung kann bspw. auf Basis einer Funktion bestimmt werden, in die (eine Ausdehnung oder) ein Integral von Abklingzeitverteilung 26" über einen Bereich NÜ, in dem keine Überlappung auftritt, eingeht. Ferner kann eine zu geringe Überlappung bspw. auf Basis einer Funktion bestimmt werden, in die ein Integral von Abklingzeitverteilung 26 und Abklingzeitverteilung 26" über Überlappungsbereich Ü eingeht.

Wie in Fig. 7 illustriert, kann die Gesamtheit aller $\tau_P$ auch zu einer Abklingzeitverteilung 26 führen, die aus mehreren symmetrischen (Gauß-)verteilungen zusammengesetzt ist. Auch hier kann eine durch Messung oder analytisch bestimmte Abklingzeitverteilung 26 als Referenz dienen und Abweichungen von der Referenz als Indizien für eine Verunreinigung interpretiert werden. Ferner kann, wie in Fig. 8a und Fig. 8b illustriert, anstatt einer statischen Referenz eine gleitende Referenz gebildet werden, was insbesondere dann vorteilhaft sein kann, wenn die Zusammensetzung der Proben (natürlichen) Schwankungen unterliegt. Liegt, wie in Fig. 9a schematisch illustriert, einer (oder mehrere) der überwachten Werte, wie bspw. eine oder mehrere charakteristische Abklingzeiten, außerhalb eines Toleranzbereiches T1 und/oder T2 um die gleitende Referenz r1 bzw. r2, kann dies als Indiz für eine Verunreinigung interpretiert werden. Wie in Fig. 9b illustriert, können auch mehrdimensionale Toleranzbereiche T1 und T2 gebildet werden.

Durch Ermitteln der Gaußverteilung 28b bewirkenden Segmente 24 kann Verunreinigung 30, wie in Fig. 10 illustriert, lokalisiert werden. Bspw. kann jedem durch einen optischen Sensor erzeugten Pixel eine Abklingzeit zugeordnet werden und es können die Pixel nach ihrer Abklingzeit gruppiert werden. Tritt eine Verunreinigung auf, kann der verunreinigte Bereich durch Pixel repräsentiert werden, die eine von einer oder mehreren typischen Abklingzeiten abweichende Abklingzeit aufweisen. Dies ermöglicht eine Lokalisierung der Verunreinigung und (bei entsprechender Auflösung) ggf. auch ein Bestimmen der Form und Größe sowie eine Identifikation der Verunreinigung.

Somit lässt sich auf Basis der Abweichungen der $\tau_P$ von einem, für eine nicht verunreinigte Probe erwarteten Wert oder Wertebereich, Verunreinigung 30 feststellen und ggf. identifizieren. Ferner kann, wie in Fig. 11 angedeutet, Rechnereinheit 18 eingerichtet sein, Probenumriss 32 zu erfassen und Verunreinigung 30 relativ zu Probenumriss 32 zu lokalisieren und zu visualisieren. Bspw. können unterschiedlichen Werten von $\tau_P$ unterschiedliche Farben oder Helligkeiten zugeordnet sein und jedes Segment 24 mit einer entsprechenden Farbe bzw. Helligkeit belegt werden. Ferner kann die Erfassung von Probenumriss 32 dazu genutzt werden, Segmente 24, die über den Rand von Probe 12 hinausgehen, zu kennzeichnen bzw. in der Ermittlung der Abklingzeitverteilung 26 nicht zu berücksichtigen.

Fig. 12 und Fig. 13 illustrieren eine Verschiebung von Abklingzeitverteilung 26 relativ zu Referenz R. Eine solche Verschiebung kann bei Lebensmitteln und insbesondere bei Fleischprodukten durch anhaltende Lichteinstrahlung verursacht werden. Somit kann aus der Verschiebung auf eine Lichteinstrahlung und somit auf die Art der Lagerung und insbesondere auf die Qualität von Probe 12 geschlossen werden. Wenn Abklingzeitverteilung 26 außerhalb des Toleranzbereiches T liegt, kann die Qualitätsverschlechterung angezeigt werden.

Fig. 14 zeigt ein Flussdiagramm eines beispielhaften Verfahrens. Das Verfahren beginnt bei Schritt 34 mit dem Bestimmen, ob Abklingzeitverteilung 26' oder 26" von einer erwarteten Abklingzeitverteilung 26 abweicht. Wenn Abklingzeitverteilung 26' bzw. 26" von der erwarteten Abklingzeitverteilung 26 abweicht, wird in Schritt 36 angezeigt, dass eine Verunreinigung vorliegt.

Fig. 15 zeigt ein Flussdiagramm eines beispielhaften Verfahrens. Das Verfahren beginnt bei Schritt 38 mit dem Erfassen von Abklingzeitverteilung 26'''. Das Verfahren wird bei Schritt 40 mit dem Bestimmen der Verschiebung der Abklingzeitverteilung 26''' relativ zur Referenz R fortgesetzt. Wenn die Abklingzeitverteilung außerhalb des Toleranzbereiches T liegt, wird in Schritt 42 die Qualitätsverschlechterung angezeigt.

Im Folgenden wird die Anwendung einiger der im Vorhergehenden beschriebenen Methoden am Beispiel von Salami aus Schweine- und Rindfleisch als typischem zerkleinertem Endprodukt dargelegt, das vielfach in Kunststoff-Verpackungen auf den Markt kommt. Bei Salami dominiert neben der Strukturierung durch die Fett-Anteile die typische rote Eigenfarbe, die teilweise durch den Zusatz von Lebensmittelfarbstoffe verstärkt wird, bspw. durch die Verwendung von natürlichem Cochenille (Karminsäure CAS-Registriernummer RN 1260-17-9), wodurch eine Charakterisierung durch Absorptionsmessungen erschwert wird. Bei der Charakterisierung mittels Fluoreszenz, bei der die fluoreszierenden Chomophore selektiert werden, können anderweitige Färbungen höchstens zur Abschwächung der Fluoreszenzintensität führen.

Fig. 16 zeigt Fluoreszenzspektrum 44 (Maximum bei 333 nm) einer naturbelassenen Salami aus Schweine- und Rindfleisch ("Bioprodukt"). Der Hauptteil des Fluoreszenzspektrums 44 erstreckt sich über den unsichtbaren UV-Bereich, mit einem schwächeren Ausläufer in den sichtbaren Bereich, der bei einer Beleuchtung mit einer starken Fluoreszenzlampe zu einem schwach bläulichen Farbton führt. Das dazugehörende Absorptionsspektrum 46 (Maximum bei 295 nm) vermittelt einen Eindruck über die Lichtabsorption von relevanten Chromophoren für die Fluoreszenz.

Wie im Folgenden an Hand von Messreihen dargestellt, ist die Fluoreszenzabklingzeit der Autofluoreszenz, deren Zeitkonstante mit hoher Präzision gemessen werden kann, charakteristisch für das Lebensmittelprodukt (hier die Salami) und deshalb für deren Identifizierung grundsätzlich von Interesse.

An sich ist es für solche Messungen naheliegend (wie auch sonst bei Fluoreszenzmessungen üblich), in das Absorptionsmaximum oder leicht kürzerwellig einzustrahlen. Das würde hier eine Fluoreszenzanregung im UV-Bereich unterhalb von 300 nm bedeuten. Manche biologische Proben absorbieren allerdings in diesem Spektralbereich sehr stark, und damit kann das Anregungslicht nicht tief in die Proben eindringen. Daher wurde zum Erreichen einer möglichst großen Eindringtiefe langwellig eingestrahlt. Bei 488 nm steht eine effiziente LASER-Emissionslinie zur Verfügung mit deren Fluoreszenzanregung eine (relativ) intensitätsstarke Fluoreszenz erhalten werden kann. Die im Folgenden diskutierten Messungen beziehen sich auf eine Fluoreszenzanregung bei dieser Wellenlänge, wobei jedoch auch andere Wellenlängen verwendet werden können. Die Messwerte wurden mit einer pco.flim Kamera aufgenommen und die Fluoreszenzabklingzeiten (bzw. Fluoreszenzabklingzeitverteilungen) wurden aus einer 2D Matrix der Größe 1008x1008 (Bildpunkte/Segmente) erhalten.

Kunststoffe können in verarbeiteten Lebensmitteln nicht nur durch Verpackungen, Transportbehälter und defekte Maschinenteile in Erscheinung treten, sondern auch durch den Eintrag als Mikroplastik in die Umwelt. Aus diesem Grund wurde mit den Kunststoffen Polyamid, Polyethylenterephthalat und Polyvinylchlorid erste Untersuchungen für drei Salamiarten durchgeführt. Als Verunreinigung wurden Kunststoff-Flakes aus PA, PET und PVC von Folien auf der Salami platziert (1-2 mm groß und mit bloßem Auge sehr schlecht bis gar nicht von Fett etc. zu unterscheiden).

Fig. 17 illustriert das Fluoreszenzabklingverhalten von Putensalami nach Anregung bei 488 nm auf Basis der Häufigkeit an Fluoreszenzabklingzeiten n als Funktion der Zeit t in ns, wobei die durchgezogene dicke Kurve die Messwerte und die dünne gestrichelte Kurve (fast vollständig durch die dicke Kurve überdeckt) einen simulierten Verlauf auf Basis einer Gaußanalyse zeigt (der Einschub zeigt eine gespreizte Darstellung des Bereichs zwischen 1 und 3 ns).

Bei hoher zeitlicher Auflösung findet man zwei ähnliche Fluoreszenzabklingzeiten, die statistisch um die Maximalwerte $\tau_1$ und $\tau_2$ streuen und in guter Näherung durch Gaußkurven ($n = n_0 \cdot \exp(-(t-\tau)^2/(2\sigma^2))$) beschrieben

werden können ($\tau_1$ = 2,004 ns, $\sigma_1$ = 0,061 ns, $n_{O1}$ = 36450; $\tau_2$ = 2,253 ns, $\sigma_2$ = 0,084 ns, $n_{O2}$ = 50025). Bei kleinerer Auflösung (0,01 ns) verschmelzen die beiden Gaußkurven zu einer. Die im Folgenden gezeigten Messungen wurden mit der kleineren Auflösung vorgenommen, da diese für die folgenden Untersuchungen und Anwendungen ausreichend ist.

Fig. 18 illustriert das Fluoreszenzabklingverhalten von Rindersalami nach Anregung bei 488 nm auf Basis der Häufigkeit an Fluoreszenzabklingzeiten n als Funktion der Zeit t in ns, wobei die durchgezogene dicke Kurve die Messwerte und die dünne gestrichelte Kurve (fast vollständig durch die dicke Kurve überdeckt) einen simulierten Verlauf auf Basis einer Gaußanalyse zeigt. Die Rindersalami weist eine charakteristische Fluoreszenzabklingzeit von $\tau_1$ = 2,60 ns, ($\sigma_1$ = 0,09 ns; $n_{O1}$ = 34100) auf.

Fig. 19 illustriert das Fluoreszenzabklingverhalten von Schweinesalami nach Anregung bei 488 nm auf Basis der Anzahl an Häufigkeit an Fluoreszenzabklingzeiten n als Funktion der Zeit t in ns, wobei die durchgezogene dicke Kurve die Messwerte und die dünne gestrichelte Kurve (fast vollständig durch die dicke Kurve überdeckt) einen simulierten Verlauf auf Basis einer Gaußanalyse zeigt. Die Schweinesalami weist eine charakteristische Fluoreszenzabklingzeit von $\tau_1$ = 2,33 ns, ($\sigma_1$ = 0,093 ns; $n_{O1}$ = 34100) auf.

Dies impliziert, dass der Anteil des roten Farbstoffes im Fleisch Auswirkung auf die Fluoreszenzabklingzeit hat (Putensalami leicht rosa, Schweinesalami rot und Rindersalami dunkelrot). Festgestellt werden kann dies über den Zusammenhang der Fluoreszenzabklingzeit und der Bestrahlungszeit, welche ein Ausbleichen des Farbstoffes hervorruft, wobei die Komponente mit der längeren Fluoreszenzabklingzeit schneller "ausbleicht" als die Komponente mit der kürzeren Fluoreszenzabklingzeit.

Fig. 20 illustriert die Änderung der Fluoreszenzabkling-Zeitkonstante $\tau$ von Putensalami als Funktion der Bestrahlungs-Dosis (Punkte). Der Zusammenhang zwischen Bestrahlungs-Dosis und Änderung kann durch eine Exponentialfunktion (erster Ordnung, dargestellt als durchgezogene Kurve) approximiert werden, wobei der Einschub eine lineare Auswertung nach erster Ordnung mit einer Steigung von 0,788 und einem Korrelationskoeffizienten von 0,995 (basierend auf 5 Messwerten) zeigt.

Wie in Fig. 20 gezeigt, kommt es abhängig von der Dosis der Lichtstrahlung durch die Mittelung (über eine Messsequenz) zu einer Verschiebung der globalen Abklingzeit zu kleineren Werten. Dieses "Bleichverhalten" kann durch eine Funktion erster Ordnung approximiert werden, wie die Linearisierung im Einschub von Fig. 20 zeigt. Für die im Folgenden beschrieben Anwendungen (zur Erkennung von Verunreinigungen) ist dies unproblematisch, weil eine einmalige Bestrahlungsdosis für eine Zuordnung des Fluoreszenzabklingverhaltens völlig ausreichend ist. Da fast alle Lebensmittel bei intensiver Sonnenbestrahlung Qualitätseinbußen erleiden, kann die Verkürzung der Fluoreszenzabklingzeit aber als Maß für die Lichteinwirkung und damit zur Qualitätsbeurteilung verwendet werden.

Die Autofluoreszenz der Salami wurde nun als Basis für die Untersuchungen von Kontaminationen (Verunreinigungen) verwendet. Wegen der Inhomogenität von Salami-Proben würde eine punktuelle Messung der Fluoreszenzabklingzeit zufällig Salami-Material oder verunreinigenden Kunststoff erfassen und wäre für die Reinheits-Kontrolle von Lebensmitteln wenig praktikabel. Die Proben, insbesondere Salamischeiben, wurden daher in der Fläche integrierend untersucht. Insbesondere wurde eine mit Polyamid verunreinigte Probe zweidimensional (d. h., die Salamischeiben wurden in der Fläche) untersucht.

Fig. 21 illustriert das Fluoreszenzabklingverhalten von Putensalami 48, welche mit Polyamid verunreinigt wurde, in einem Randbereich 52 der Verunreinigung 50. Zu erkennen ist ein für Putensalami 48 charakteristisches Maximum 56a bei $\tau_1$ = 2,21 ns ($\sigma_1$ = 0,075 ns, $n_{O1}$ = 80000) und ein weiteres für das verunreinigende Polyamid charakteristische Maximum 56b bei $\tau_2$ = 3,28 ns ($\sigma_2$ = 0,14 ns, $n_{O2}$ = 27900), durch das die Verunreinigung eindeutig identifiziert werden kann. Ein drittes weniger stark ausgeprägtes Maximum 56c mit $\tau_3$ = 2,91 ns ($\sigma_3$ = 0,27 ns, $n_{O3}$ = 20500) tritt zwischen den charakteristischen Abklingzeiten von Polyamid und Putensalami 48 auf. Dieses kommt dadurch zustande, dass sich Kunststoff und Putensalami 48 in Randbereich 52 überlappen, was zu einer Überlagerung und Konvolution der beiden Fluoreszenzabklingzeitsignale führt. Wie in Fig. 21 angedeutet, lässt sich das Abklingzeitspektrum 54 in drei symmetrische (Gauß-)verteilungen um die Maxima 56a, 56b und 56c zerlegen.

Fig. 22 illustriert das Fluoreszenzabklingverhalten von Putensalami 48, welche mit PVC verunreinigt wurde, in einem Randbereich 52 der Verunreinigung 50. Auch hier existiert neben dem für Putensalami 48 charakteristischen Maximum 56a bei $\tau_1$ = 2,62 ns ($\sigma_1$ = 0,12 ns, $n_{O1}$ = 172000) ein weiteres für das verunreinigende PVC charakteristisches Maximum 56b bei $\tau_2$ = 5,42 ns ($\sigma_2$ = 0,18 ns, $n_{O2}$ = 60000), wodurch die Verunreinigung eindeutig identifiziert werden kann. Ebenso findet sich auch ein drittes Maximum 56c bei $\tau_3$ = 4,38 ns ($\sigma_3$ = 0,18 ns, $n_{O3}$ = 18600), welche durch die Überlagerung der beiden Fluoreszenzabklingzeitsignale in Randbereich 52 hervorgerufen werden.

Fig. 23 illustriert das Fluoreszenzabklingverhalten von Putensalami 48, welche mit PET verunreinigt wurde, in einem Randbereich der Verunreinigung 50. Da hier die Fluoreszenzabklingzeiten von Putensalami 48 und Verunreinigung 50 ähnlicher sind, findet man zwei relativ nahe beieinander liegende Maxima 56a und 56b. Eine Gaußanalyse ergibt zwei Maxima bei $\tau_1$ = 2,33 ns ($\sigma_1$ = 0,074 ns, $n_{O1}$ = 250000) und $\tau_2$ = 2,50 ns ($\sigma_2$ = 0,069 ns, $n_{O2}$ = 19501). Das zeigt, dass die Analyse auch auf Fälle anwendbar ist, in denen die Fluoreszenzabklingzeit der Verunreinigung nahe an der Fluoreszenzabklingzeit der Verunreinigung liegt.

Fig. 24 illustriert das Fluoreszenzabklingverhalten von Putensalami 48, welche auf einer der Einstrahlung gegenüberliegenden Seite mit Polyamid verunreinigt wurde. Auch hier kommt es zu einer Verschiebung von Maximum 56a bei $\tau$ = 2,92 ns ($\sigma$ = 0,12 ns, $n_O$ = 16950). Somit ist die Kontamination wiederum eindeutig feststellbar, da es zu einer Faltung der Fluoreszenzabklingzeiten von Polyamid und der Salami kommt, wobei sich die höhere Eindringtiefe des anregenden Lichts positiv auswirkt.

Da ein möglicher Eintrag in verarbeitete Lebensmittel durch Abriebe oder Bruchstücke von Transportbehältern vorkommen kann und rote Eurobehälter E2 aus HDPE zugelassene Transportbehälter in fleischverarbeitenden Betrieben sind, wurden Messungen mit Schweinesalami und Rindersalami durchgeführt, die zeigen, dass sich Späne und Bruchstücke (in der Untersuchung ca. 2 mm groß), auf, hinter und zwischen Salamischeiben detektieren lassen.

Dazu wurden Bruchstücke und Späne eines Euronorm E2 Behälters, welcher zum Fleischtransport in Fleischherstellungsbetrieben verwendet wird, auf und unter einer Salamischeibe, sowie zwischen zwei Salamischeiben platziert. Zur Inspektion der Proben stand ein Mikroskop mit einem zwanzigfachen Vergrößerungsobjektiv der Firma Nikon zur Verfügung, an welchem auch der Laser und die Kamera befestigt waren. Die Salamiproben wurden auf einem gläsernen Objektträger mit den Kunststoffen präpariert.

Fig. 25 illustriert das Fluoreszenzabklingverhalten von Rindersalami 48, welche mit einem Kunststoff verunreinigt wurde, in einem Randbereich 52 der Verunreinigung 50. Fluoreszenzabklingspektrum 54 weist ein für die Rindersalami charakteristisches Maximum 56a bei $\tau_1$ = 2,465 ns ($\sigma_1$ = 0,09 ns, $n_{O1}$ = 45900), ein Maximum 56c der konvolutionierten Fluoreszenzabklingzeit, die sich im Überlappungsbereich 52 zwischen dem Kunststoff und Rindersalami 48 einstellt, bei $\tau_2$ = 2,8 ns ($\sigma_2$ = 0,14 ns, $n_{O2}$ = 58000) und ein für den Kunststoff des roten Eurobehälters HDPE charakteristisches Maximum bei $\tau_3$ = 3,84 ns ($\sigma_3$ = 0,10 ns, $n_{O3}$ = 18000) auf. Die relativ schwache Ausprägung von Maximum 56b liegt an der geringen Größe, die der Kunststoff in der 1008×1008 Matrix einnimmt.

Fig. 26 illustriert das Fluoreszenzabklingverhalten von Rindersalami 48, welche mit Kunststoff auf einer der Einstrahlung gegenüberliegenden Seite verunreinigt wurde. Dabei wurde ein Maximum 56c der Mischabklingzeit für Verunreinigung 50 und Rindersalami 48 bei $\tau_1$ = 2,91 ns ($\sigma_1$ = 0,12 ns, $n_{O1}$ = 77600) gemessen. Darüber hinaus existiert ein für den Kunststoff charakteristisches Maximum 56b bei $\tau_2$ = 3,72 ns ($\sigma_2$ = 0,13 ns, $n_{O2}$ = 31500).

Fig. 27 illustriert das Fluoreszenzabklingverhalten von zwei Rindersalamischeiben 48, zwischen denen ein Kunststoffstück angeordnet ist. Dabei wurde nur Maximum 56c bei $\tau$ = 3,15 ns ($\sigma$ = 0,12 ns, $n_O$ = 37450) erfasst, was auf eine Mischabklingzeit schließen lässt, die im Vergleich zur Abklingzeit der Rindersalami verschoben ist. Somit belegen die in Fig. 25. Fig. 26 und Fig. 27 gezeigten Messungen, dass Kunststoff sowohl auf als auch unter und zwischen Salamischeiben detektiert werden kann.

Fig. 28 illustriert das Fluoreszenzabklingverhalten von Schweinesalami 48, welche mit einem Kunststoff verunreinigt wurde, in einem Randbereich 52 der Verunreinigung 50. Dabei ergeben sich, Maxima 56a, 56b und 56c, wobei das charakteristische Maximum 56a der Schweinesalami 48 bei $\tau_1$ = 2,36 ns ($\sigma_1$ = 0,19 ns, $n_{O1}$ = 56000) liegt. Das Maximum 56c bei $\tau_2$ = 3,2 ns ($\sigma_2$ = 0,40 ns, $n_{O2}$ = 14200) spiegelt die Mischabklingzeit wider, die sich aus der Konvolution der Fluoreszenzsignale des Kunststoffes und der Schweinesalami 48 zusammensetzt. Das Maximum 56b bei $\tau_3$ = 4,03 ns ($\sigma_3$ = 0,08 ns, $n_{O3}$ = 20000) ist charakteristisch für den Kunststoff.

Fig. 29 illustriert das Fluoreszenzabklingverhalten von Schweinesalami 48, welche mit Kunststoff auf einer der Einstrahlung gegenüberliegenden Seite verunreinigt wurde. Dabei werden zwei dicht beieinander liegende Maxima 56a und 56b beobachtet, die jedoch voneinander unterschieden werden können. Maximum 56a bei $\tau_1$ = 2,44 ns ($\sigma_1$ = 0,14 ns, $n_{O1}$ = 43100) ist charakteristisch für die Schweinesalami 48 und das Maximum 56b bei $\tau_2$ = 2,73 ns ($\sigma_2$ = 0,065 ns, $n_{O2}$ = 21000) repräsentiert die Mischabklingzeit aus dem Fluoreszenzsignal der Schweinesalami 48 und dem der Verunreinigung 50.

Fig. 30 illustriert das Fluoreszenzabklingverhalten von zwei Schweinesalamischeiben, zwischen denen ein Kunststoffstück angeordnet ist. Dabei zeigt sich, dass der Kunststoff zwischen den Scheiben einfacher detektiert werden kann, als wenn er rückseitig auf nur einer Scheibe angebracht ist, da wegen der zweiten Scheibe eine weitere Reflexionsfläche hinzukommt, die mehr Fluoreszenzlicht auf den Matrixdetektor zurückwirft. Es können drei Maxima 56a, 56b und 56c ermittelt werden. Das Maximum 56a bei $\tau_1$ = 2,485 ns ($\sigma 1$ = 0,19 ns, $n_{O1}$ = 56000), das für die Schweinesalami 48 charakteristisch ist, ein Maximum 56c bei $\tau_2$ = 3,74 ns ($\sigma_2$ = 0,22 ns, $n_{O2}$ = 20600), das aus der Überlappung der Abklingzeiten des Kunststoffes und der Abklingzeit der Salami resultiert und ein drittes Maximum 56b bei $\tau_3$ = 3,98 ns ($\sigma_3$ = 0,066 ns, $n_{O3}$ = 20000), das die Verunreinigung 50 repräsentiert.

Fig. 31 illustriert das Fluoreszenzspektrum eines Salatblatts bei einer Anregungswellenlänge von 488 nm. Das Fluoreszenzspektrum des Salatblatts zeigt eine Stokes-Verschiebung von >200 nm, wohingegen der verwendete Kunststoff Kunststoffe mit einer Stokes-Verschiebung von 20-100 nm fluoresziert. Wird im Strahlengang ein optischer Bandpassfilter eingesetzt, kann somit erreicht werden, dass nur das Fluoreszenzsignal des Kunststoffes aufgenommen wird. So zeigt die durchgezogene Linie in Fig. 31 das Fluoreszenzspektrum ohne Bandpassfilter und die gestichelte Linie das Fluoreszenzspektrum von Salat mit einem optischen Bandpassfilter (Cut-Off-Wellenlänge = 550 nm).

Fig. 32 illustriert das Fluoreszenzabklingverhalten eines Salatblattes ohne Verunreinigung, wobei die durchgezogene, dicke Kurve die experimentellen Werte und die gestichelte, dünne Kurve (fast vollständig vom experimentellen Verlauf überdeckt) einen simulierten Verlauf auf der Basis einer Gaußanalyse repräsentiert. Die Messung zeigt ein Maximum der Fluoreszenzabklingzeitverteilung bei: ($\tau_1$ = 1,81 ns, $\sigma_1$ = 0,09 ns, $n_{O1}$ = 39615). Diese muss berücksichtigt werden, da das Fluoreszenzsignal (die Fluoreszenzabklingzeit), obwohl es am Detektor (auf Grund der optischen Blockung durch den optischen Bandpassfilter) nicht registriert wird, einen Einfluss auf das Fluoreszenzsignal des Kunststoffes hat.

Fig. 33 illustriert das Fluoreszenzabklingverhalten eines Salatblattes, welches auf der Oberseite mit rotem HDPE Kunststoff verunreinigt wurde, wobei die durchgezogene, dicke Kurve die experimentellen Werte und die gestichelte, dünne Kurve (größtenteils vom experimentellen Verlauf überdeckt) einen simulierten Verlauf auf der Basis einer Gaußanalyse repräsentiert. Die Messung zeigt ein geringfügig verschobenes Maximum der Fluoreszenzabklingzeitverteilung des Kunststoffes bei: $\tau_1$ = 3,35 ns, $\sigma_1$ = 0,26 ns, $n_{O1}$ = 68244, durch das die Verunreinigung eindeutig erfasst werden kann.

Fig. 34 illustriert das Fluoreszenzabklingverhalten eines Salatblattes, welches auf der Unterseite mit rotem HDPE Kunststoff verunreinigt wurde, wobei die durchgezogene, dicke Kurve die experimentellen Werte und die gestichelte, dünne Kurve (teilweise vom experimentellen Verlauf überdeckt) einen simulierten Verlauf auf der Basis einer Gaußanalyse repräsentiert. Dabei wird zwar kein Fluoreszenzsignal des Salats detektiert, allerdings transmittiert die Fluoreszenz des Kunststoffes durch das Salatblatt hindurch und wird dadurch beeinflusst. Der Detektor erfasst eine Fluoreszenzabklingzeit von: $\tau_1$ = 2,54 ns, $\sigma_1$ = 0,59 ns, $n_{O1}$ = 31107, wobei das Maximum auf eine Mischabklingzeit aus den Komponenten Kunststoff und Salat hinweist. Wiederum kann der Kunststoff eindeutig detektiert werden.

Fig. 35 illustriert das Fluoreszenzabklingverhalten eines Salatbreis, welcher auf der Oberseite mit rotem HDPE Kunststoff verunreinigt wurde, wobei die durchgezogene, dicke Kurve die experimentellen Werte und die gestichelte, dünne Kurve (teilweise vom experimentellen Verlauf überdeckt) einen simulierten Verlauf auf der Basis einer Gaußanalyse repräsentiert. Das Maximum der Fluoreszenzabklingzeitverteilung liegt bei: $\tau_1$ = 3,47 ns, $\sigma_1$ = 0,3 ns, $n_{O1}$ = 49192 und ist trotz des Einflusses des Salats charakteristisch für die Verunreinigung, die dadurch eindeutig erfasst werden kann.

Fig. 36 illustriert das Fluoreszenzabklingverhalten eines Salatbreis, welcher unter einer dünnen Schicht mit dem roten HDPE Kunststoff verunreinigt wurde, wobei die durchgezogene, dicke Kurve die experimentellen Werte und die gestichelte, dünne Kurve (größtenteils vom experimentellen Verlauf überdeckt) einen simulierten Verlauf auf der Basis einer Gaußanalyse repräsentiert. Die Verunreinigung bewirkte ein verschobenes Ma-

ximum der Fluoreszenzabklingzeitverteilung bei: $\tau_1$ = 3,27 ns, $\sigma_1$ = 0,52 ns, $n_{O1}$ = 21927, wodurch eine Detektion des Kunststoffes ermöglicht wird.

Fig. 37 illustriert das Fluoreszenzabklingverhalten von Schinken, welcher nicht verunreinigt wurde, wobei die durchgezogene, dicke Kurve die experimentellen Werte und die gestichelte, dünne Kurve (teilweise vom experimentellen Verlauf überdeckt) einen simulierten Verlauf auf der Basis einer Gaußanalyse repräsentiert. Das Maximum der Fluoreszenzabklingzeitverteilung von Schinken ergibt sich nach der Gaußanalyse bei: $\tau_1$ = 1,60 ns, $\sigma_1$ = 0,66 ns, $n_{O1}$ = 88458. Dabei zeigte sich, dass Schinken weniger intensiv fluoresziert als die Salami. Bei der Messung wurde daher eine relativ hohe Belichtungszeit eingestellt, wodurch die Standardabweichung größer ist als bei den Messungen mit Salami.

Fig. 38 illustriert das Fluoreszenzabklingverhalten von Schinken, welcher mit rotem HDPE Kunststoff auf der Oberfläche verunreinigt wurde, wobei die durchgezogene, dicke Kurve die experimentellen Werte und die gestichelte und gepunktete, dünne Kurve einen simulierten Verlauf auf der Basis einer Gaußanalyse repräsentiert. Die Maxima der Gaußkurven 56a und 56b beschreiben die charakteristischen, zueinander verschobenen Maxima der Fluoreszenzabklingzeitverteilung des Schinkens ($\tau_1$ = 2,50 ns, $\sigma_1$ = 0,7 ns, $n_{O1}$ = 12900) und der Kunststoffverunreinigung ($\tau_2$ = 3,42 ns, $\sigma_2$ = 0,23 ns, $n_{O2}$ = 39500). Der Kunststoff kann dadurch eindeutig detektiert und identifiziert werden.

Fig. 39 illustriert das Fluoreszenzabklingverhalten von Schinken, welcher mit rotem HDPE Kunststoff an der Unterseite verunreinigt wurde, wobei die durchgezogene, dicke Kurve die experimentellen Werte und die gestichelte, dünne Kurve (teilweise vom experimentellen Verlauf überdeckt) einen simulierten Verlauf auf der Basis einer Gaußanalyse repräsentiert. Dabei wurde eine Mischfluoreszenzabklingzeit von: $\tau_1$ = 2,38 ns, $\sigma_1$ = 0,65 ns, $n_{O1}$ = 29668 erfasst, wobei das Maximum auf eine Mischabklingzeit aus den Komponenten Kunststoff und Schinken hinweist. Somit kann der Kunststoff eindeutig unter dem Schinken detektiert werden.

Fig. 40 illustriert das Fluoreszenzabklingverhalten von Fisch, wobei die durchgezogene, dicke Kurve die experimentellen Werte und die gestichelte, dünne Kurve (teilweise vom experimentellen Verlauf überdeckt) einen simulierten Verlauf auf der Basis einer Gaußanalyse repräsentiert. Das verwendete Fischfilet weist ein charakteristisches Maximum der Fluoreszenzabklingzeitverteilung bei: $\tau_1$ = 1,48 ns, $\sigma_1$ = 0,54 ns, $n_{O1}$ = 102288 auf. Auch hier zeigt sich, dass durch eine höhere Belichtungszeit, da die Fluoreszenz des Fisches gering ist, die Standardabweichung größer ist als bspw. bei Salami.

Fig. 41 illustriert das Fluoreszenzabklingverhalten von Fisch, welcher an der Oberfläche mit HDPE Kunststoff verunreinigt wurde, wobei die durchgezogene, dicke Kurve die experimentellen Werte und die gestichelte und die gepunktete, dünne Kurve einen simulierten Verlauf auf der Basis einer Gaußanalyse repräsentiert. Der mit einem Kunststoffpartikel verunreinigte Fisch zeigt in seiner Auswertung zwei Fluoreszenzmaxima. Dabei beschreibt 56a das verschobene Fluoreszenzmaximum des Fisches mit: ($\tau_1$ = 2,30 ns, $\sigma_1$ = 0,7 ns, $n_{O1}$ = 35000) und 56b das verschobene Fluoreszenzmaximum des Kunststoffes mit: ($\tau_2$ = 3,25 ns, $\sigma_2$ = 0,4 ns, $n_{O2}$ = 105000). Der Kunststoff kann eindeutig auf dem Fisch detektiert und identifiziert werden.

Fig. 42 illustriert das Fluoreszenzabklingverhalten von Fisch, welcher im Inneren mit rotem HDPE Kunststoff verunreinigt wurde, wobei die durchgezogene, dicke Kurve die experimentellen Werte und die gestichelte, dünne Kurve (teilweise vom experimentellen Verlauf überdeckt) einen simulierten Verlauf auf der Basis einer Gaußanalyse repräsentiert. Die Messungen an Fisch, welcher im Inneren, unter einer ca. 1 mm dicken Fischschicht, mit Kunststoff verunreinigt wurde, zeigen eine Verschiebung des Fluoreszenzmaximums zu: $\tau_1$ = 2,05 ns, $\sigma_1$ = 1 ns, $n_{O1}$ = 33800. Es lässt sich eine Verschiebung der charakteristischen Fluoreszenzabklingzeit des Fisches um 0,5 ns feststellen, was eine Detektion des Kunststoffes möglich macht.

Fig. 43 illustriert das Fluoreszenzabklingverhalten von Honig, wobei die durchgezogene, dicke Kurve die experimentellen Werte und die gestichelte, dünne Kurve (fast vollständig vom experimentellen Verlauf überdeckt) einen simulierten Verlauf auf der Basis einer Gaußanalyse repräsentiert. Die Fluoreszenzabklingzeitverteilung hat ein Maximum bei: $\tau_1$ = 1,91 ns, $\sigma_1$ = 0,12 ns, $n_{O1}$ = 65408.

Fig. 44 illustriert das Fluoreszenzabklingverhalten von Honig, welcher eine Verunreinigung von rotem HDPE an der Oberfläche aufweist, wobei die durchgezogene, dicke Kurve die experimentellen Werte und die gestichelte, dünne Kurve (fast vollständig vom experimentellen Verlauf überdeckt) einen simulierten Verlauf auf der Basis einer Gaußanalyse repräsentiert. Dabei ergibt sich eine Fluoreszenzabklingzeitverteilung mit einem Maximum bei: $\tau_1$ = 2,75 ns, $\sigma_1$ = 0,19 ns, $n_{O1}$ = 10122. Es wird also eine Mischabklingzeit aus Honig und dem roten HDPE detektiert, wodurch die Verunreinigung erkannt werden kann.

Fig. 45 illustriert das Fluoreszenzabklingverhalten von Honig, welcher eine Verunreinigung mit rotem HDPE 3 mm unter der Oberfläche aufweist, wobei die durchgezogene, dicke Kurve die experimentellen Werte und die gestichelte, dünne Kurve (fast vollständig vom experimentellen Verlauf überdeckt) einen simulierten Verlauf auf der Basis einer Gaußanalyse repräsentiert. Dabei wurde ein Fluoreszenzmaximum bei: $\tau_1$ = 2,34 ns, $\sigma_1$ = 0,13 ns, $n_{O1}$ = 15371 detektiert. Somit kann auch hier die Verunreinigung eindeutig detektiert werden.

Fig. 46 illustriert das Fluoreszenzabklingverhalten von Honig, welcher eine Verunreinigung mit rotem HDPE 5 mm unter der Oberfläche aufweist, wobei die durchgezogene, dicke Kurve die experimentellen Werte und die gestichelte, dünne Kurve (fast vollständig vom experimentellen Verlauf überdeckt) einen simulierten Verlauf auf der Basis einer Gaußanalyse repräsentiert. Es ergibt sich auch hier eine Mischabklingzeit, wobei ein Maximum bei: $\tau_1$ = 2,17 ns, $\sigma_1$ = 0,11 ns, $n_{O1}$ = 18208 detektiert wird. Auch hier ist eine Verschiebung der Fluoreszenzabklingzeit zu sehen. Durch die Zunahme der Honigschicht, wird der Einfluss der Fluoreszenzabklingzeit von Honig größer. Trotzdem ist eine Detektion des Partikels unter einer 5 mm dicken Schicht von Honig möglich.

Fig. 47 illustriert Systeme 58 zur Kontrolle und Detektion von Verunreinigungen in Lebensmitteln (bspw. durch Kunststoffe in der Lebensmittelverarbeitung). Jedes System 58 umfasst Lichtquelle 60, welche ein Laser, eine Laserdiode, eine LED oder ein ähnlicher strahlender Gegenstand sein kann, der die Autofluoreszenz hervorruft. Lichtquelle 60 emittiert Fluoreszenzanregungslicht 62 auf Lebensmittel 64 (hier als Wurstscheiben schematisch dargestellt) und Fremd-Kunststoff 66, welcher sich auf, unter oder zwischen dem Lebensmittel bzw. den Lebensmitteln befindet.

Die Lebensmittel 64 und der Fremd-Kunststoff 66 befinden sich bei einem In-Line Prozess auf einem Förderband 68. Das dadurch hervorgerufene Autofluoreszenzsignal 70 des Lebensmittels 64 bzw. des Kunststoffes 66 strahlt zurück auf eine Detektionseinheit 72, welches eine Kamera, eine Photodiode, oder ähnliches sein kann, was Lichtsignale, insbesondere Fluoreszenzsignale, Phasenverschiebungen oder Modulationsveränderungen messen kann, die zur Berechnung der Fluoreszenzabklingzeit-Konstante benötigt werden. Der gezeigte Auswertealgorithmus für die gemessenen Daten kann von der im Hintergrund arbeitenden Rechnereinheit (nicht gezeigt) durchgeführt werden, die darüber hinaus für die zeitliche Synchronisation zwischen Lichtquelle 60 und Detektionseinheit 166 sorgen kann.

Das im Vorhergehenden beschriebe Verfahren erlaubt die Detektion von Plastik-Verunreinigungen, insbesondere Polyamid, Polyethylen, Polyvinylchlorid, Polyethylenterephthalat, Polystyrol, Polypropylen, ABS-Kunststoffe, in Lebensmitteln (insbesondere Fleischprodukten wie Wurst und bspw. Salami) unter Verwendung der Fluoreszenzabklingzeit der Autofluoreszenz und deren charakteristischen Abklingzeit-Konstante.

Bevorzugtes Fleisch für Fleischprodukte ist Schweinefleisch, Rindfleisch, Schaffleisch, Ziegenfleisch und auch weniger geläufiges Fleisch wie Wild - hier Wildschwein-, Reh-, Hirsch- oder Hasenfleisch oder auch Kaninchenfleisch-, Antilopen- und Känguruhfleisch oder aber auch Geflügelfleisch wie Truthahn-, Hühner-, Gänse-, Enten- oder Taubenfleisch oder aber auch Fisch-Fleisch wie Fleisch von Forellen, Fellchen, Barsch, Lachs, Kabeljau, Rotbarsch, Makrele, Scholle, Heilbutt.

Zur Fluoreszenzanregung kann Laserlicht verwendet werden, bevorzugt Laserlicht mit einer Wellenlänge von 488 nm.

Zur Fluoreszenzanregung kann das Laserlicht in die Vorderseite oder in die Rückseite von Proben mit offenen oder verdeckten Plastik-Verunreinigungen eingestrahlt wird, bevorzugt in die Vorderseite.

Zur Fluoreszenzanregung kann ferner moduliertes Laserlicht verwendet und die Phasenverschiebung des Fluoreszenzsignals gemessen werden.

Das im Vorhergehenden beschriebene Verfahren kann ferner zur Qualitätskontrolle von Lebensmitteln (bspw. Fleischprodukten) eingesetzt werden, insbesondere zur Beurteilung der Einwirkung von Licht durch die Bestimmung der Lage des Maximums der Fluoreszenzabkling-Zeitkonstante.

Das Ergebnis kann bspw. in einem Zeigerdiagramm dargestellt werden.

Wegen der kurzen Zeitkonstante des Fluoreszenzabklingvorgangs von wenigen Nanosekunden und der Zeit, Daten zu akquirieren (man kann hier eine maximale Dauer von 10 Zeitkonstanten annehmen), kann die komplette Erkennung jeweils innerhalb von 50 bis 100 ns ablaufen. Dadurch lassen sich sehr hohe Verarbeitungsgeschwindigkeiten realisieren, die kaum durch das optische Detektionsverfahren begrenzt werden. Dies ermöglicht einen breiten Einsatz des Verfahrens für diverse technologische Anwendungen.

Zudem ist anzumerken, dass Kunststoffe in verarbeiteten Lebensmitteln nicht nur durch Verpackungen, Transportbehälter und defekte Maschinenteile in Erscheinung treten können, sondern auch durch den Eintrag als Mikroplastik in die Umwelt. Das gezeigte Verfahren ist für die Erkennung von Mikroplastik in Lebensmitteln geeignet, wohingegen Mikroplastik auf Grund der kleinen Dimensionen mit anderen Methoden erheblich schwieriger zu detektieren ist.

BEZUGSZEICHENLISTE

| | |
|---|---|
| 10 | Gerät |
| 10a | Anzeigeeinrichtung |
| 10b | Aussortiereinrichtung |
| 10c | Reinigungseinrichtung |
| 10d | Schneidemaschine |
| 12 | Probe |
| 14 | Emitter |
| 16 | Detektor |
| 18 | Rechnereinheit |
| 20 | Amplitudenmodulation |
| 22 | Amplitudenmodulation |
| 24 | Segment |
| 26 | Abklingzeitverteilung |
| 26' | Abklingzeitverteilung |
| 26" | Abklingzeitverteilung |
| 26''' | Abklingzeitverteilung |
| 28a | Verteilung |
| 28b | Verteilung |
| 28c | Verteilung |
| 30 | Verunreinigung |
| 32 | Probenumriss |
| 34 | Schritt |

| 36 | Schritt |
|---|---|
| 38 | Schritt |
| 40 | Schritt |
| 42 | Schritt |
| 44 | Fluoreszenzspektrum |
| 46 | Anregungsspektrum |
| 48 | Salami |
| 50 | Verunreinigung |
| 52 | Randbereich |
| 54 | Abklingzeitverteilung |
| 56a | (lokales) Maximum |
| 56b | (lokales) Maximum |
| 56c | (lokales) Maximum |
| 58 | System |
| 60 | Lichtquelle |
| 62 | Fluoreszenzanregungslicht |
| 64 | Lebensmittel |
| 66 | Kunststoff |
| 68 | Förderband |
| 70 | Autofluoreszenzsignal |
| 72 | Detektionseinheit |
| 100 | Fördersystem |
| D | Abstand |
| D1 | Abstand |
| D2 | Abstand |
| M1 | (lokales) Maximum |
| M2 | (lokales) Maximum |
| M3 | (lokales) Maximum |
| R | Referenz |
| R' | Referenz |
| $t1$ | charakteristische Abklingzeit |
| $t2$ | charakteristische Abklingzeit |
| $s1$ | charakteristische Anzahl an Segmenten |
| $s2$ | charakteristische Anzahl an Segmenten |
| T | Toleranzbereich |
| T' | Toleranzbereich |
| T1 | Toleranzbereich |
| T2 | Toleranzbereich |
| Ü | Bereich mit Überlappung |
| NÜ | Bereich ohne Überlappung |
| NÜ1 | Bereich ohne Überlappung |
| NÜ2 | Bereich ohne Überlappung |

**Patentansprüche**

1. Verfahren zur Überprüfung einer Probe (12) hinsichtlich einer Verunreinigung (30), umfassend:

   Bestimmen einer Abweichung einer Abklingzeitverteilung (26) einer durch eine elektromagnetische Strahlung angeregten Autofluoreszenz der Probe (12) von einer erwarteten Abklingzeitverteilung (R);
   **dadurch gekennzeichnet, dass**
   die Verunreinigung (30) Kunststoff, Glas, Metall oder Keramik umfasst oder aus Kunststoff, Glas, Metall oder Keramik besteht.

2. Verfahren zur Überprüfung einer Probe (12) nach Anspruch 1, ferner umfassend:
   Anzeigen (36) einer Verunreinigung (30) der Probe (12), wenn die Abweichung der Verunreinigung (30) zugeordnet ist.

3. Verfahren zur Überprüfung einer Probe (12) nach Anspruch 1 oder 2, ferner umfassend: Zerlegen der Abklingzeitverteilung (26) der durch die elektromagnetische Strahlung angeregten Autofluoreszenz der Probe (12) in einen oder mehrere einem Material der Probe (12) zugeordnete Anteile und einen oder mehrere Restanteile.

4. Verfahren zur Überprüfung einer Probe (12) nach Anspruch 3, ferner umfassend: Abgleichen des einen oder der mehreren dem Material der Probe (12) zugeordneten Anteile mit einer charakteristischen Abklingzeitverteilung eines Nahrungsmittels.

5. Verfahren zur Überprüfung einer Probe (12) nach einem der Ansprüche 1 bis 4, wobei die elektromagnetische Strahlung eine Wellenlänge im Bereich von 425 nm bis 550 nm, vorzugsweise 488 nm, aufweist.

6. Verfahren zur Überprüfung einer Probe (12) nach einem der Ansprüche 3 bis 5, ferner umfassend: Abgleichen des einen oder der mehreren Restanteile mit einer Abklingzeitverteilung der Verunreinigung (30).

7. Verfahren zur Überprüfung einer Probe (12) nach Anspruch 1, wobei der Kunststoff Polyamid, Polyethylen, Polyvinylchlorid, Polyethylenterephthalat, Polystyrol, Polypropylen, oder Acrylnitril-Butadien-Styrol umfasst.

8. Verfahren zur Überprüfung einer Probe (12) nach einem der Ansprüche 1 bis 7, ferner umfassend: Vergleichen von Abklingzeitverteilungen (26) der durch die elektromagnetische Strahlung angeregten Autofluoreszenz von zwei oder mehr unterschiedlichen Probenvolumina oder Proben (12), wobei die Verunreinigung (30) angezeigt wird, wenn eine Abweichung der zwei oder mehr Abklingzeitverteilungen (26) voneinander außerhalb eines Toleranzbereichs liegt.

9. Verfahren zur Überprüfung einer Probe (12) nach Anspruch 1, ferner umfassend:

   Bestimmen (40) einer Verschiebung der Abklingzeitverteilung (26) relativ zu einer Referenz (R); und
   Anzeigen (42) einer Qualitätsverschlechterung, wenn die Abklingzeitverteilung (26) außerhalb eines Toleranzbereichs (T) um die Referenz (R) liegt.

10. Gerät (10) zur Überprüfung einer Probe (12) hinsichtlich einer Verunreinigung (30), umfassend:

einen Emitter (14), eingerichtet zur Aussendung einer frequenz- oder amplitudenmodulierten elektromagnetischen Strahlung; und einen Detektor (16), eingerichtet zur Aufnahme einer durch die elektromagnetische Strahlung angeregten Autofluoreszenz der Probe (12); wobei das Gerät (10) eingerichtet ist, ein Ergebnis der Überprüfung auf Basis einer Abklingzeitverteilung (26) der Autofluoreszenz der Probe (12) zu bestimmen; wobei das Gerät (10) ferner eingerichtet ist, eine Verunreinigung (30) der Probe (12) anzuzeigen, wenn die Abklingzeitverteilung (26) der Autofluoreszenz der Probe (12) von einer erwarteten Abklingzeitverteilung (R) abweicht; **dadurch gekennzeichnet, dass**

die Verunreinigung (30) Kunststoff, Glas, Metall oder Keramik umfasst oder aus Kunststoff, Glas, Metall oder Keramik besteht.

11. Gerät (10) zur Überprüfung einer Probe (12) nach Anspruch 10, wobei das Gerät (10) ferner eingerichtet ist, einen Probenumriss (32) zu erfassen und die Verunreinigung (30) relativ zum Probenumriss (32) zu lokalisieren.

12. Gerät (10) zur Überprüfung einer Probe (12) nach Anspruch 10 oder 11, wobei das Gerät (10) ferner eingerichtet ist, eine Qualität der Probe (12) zu beanstanden, wenn eine Abklingzeit der Autofluoreszenz geringere Werte aufweist, als erwartet.

13. Gerät (10) zur Überprüfung einer Probe (12) nach einem der Ansprüche 10 bis 12, ferner umfassend: ein Messer, wobei das Gerät (10) zum Zerteilen eines Lebensmittels eingerichtet ist.

**Claims**

1. Method for inspecting a sample (12) with respect to a contamination (30), comprising:

determining a deviation of a decay time distribution (26) of an autofluorescence of the sample (12) excited by an electromagnetic radiation, from an expected decay time distribution (R); **characterised in that** the contamination (30) comprises plastic, glass, metal or ceramic or consists of plastic, glass, metal or ceramic.

2. Method for inspecting a sample (12) according to claim 1, further comprising: indicating (36) a contamination (30) of the sample (12) if the deviation is associated with the contamination (30).

3. Method for inspecting a sample (12) according to claim 1 or 2, further comprising: separating the decay time distribution (26) of the autofluorescence of the sample (12) excited by the electromagnetic radiation into one or more fractions associated with a material of the sample (12) and one or more residual fractions.

4. Method for inspecting a sample (12) according to claim 3, further comprising: comparing the one or more fractions associated with the material of the sample (12) with a characteristic decay time distribution of a foodstuff.

5. Method for inspecting a sample (12) according to any one of claims 1 to 4, wherein the electromagnetic radiation has a wavelength in the range of 425 nm to 550 nm, preferably 488 nm.

6. Method for inspecting a sample (12) according to any one of claims 3 to 5, further comprising: comparing the one or more residual fractions with a decay time distribution of the contamination (30).

7. Method for inspecting a sample (12) according to claim 1, where the plastic comprises polyamide, polyethylene, polyvinyl chloride, polyethylene terephthalate, polystyrene, polypropylene or acrylonitrile-butadiene-styrene.

8. Method for inspecting a sample (12) according to any one of claims 1 to 7, further comprising: comparing decay time distributions (26) of the autofluorescence excited by the electromagnetic radiation of two or more different sample volumes or samples (12), wherein the contamination (30) is indicated if a deviation of the two or more decay time distributions (26) from one another is outside a tolerance range.

9. Method for inspecting a sample (12) according to claim 1, further comprising:

determining (40) a shift in the decay time distribution (26) relative to a reference (R); and indicating (42) a deterioration in quality if the decay time distribution (26) is outside a tolerance range (T) around the reference (R).

10. Appliance (10) for inspecting a sample (12) with respect to a contamination (30), comprising:

an emitter (14) designed to emit frequency- or amplitude-modulated electromagnetic radiation; and

a detector (16) designed to receive an autofluorescence from the sample (12) excited by the electromagnetic radiation;

wherein the appliance (10) is designed to determine a result of the inspection on the basis of a decay time distribution (26) of the autofluorescence of the sample (12);

wherein the appliance (10) is further designed to indicate a contamination (30) of the sample (12) if the decay time distribution (26) of the autofluorescence of the sample (12) deviates from an expected decay time distribution (R); **characterised in that**

the contamination (30) comprises plastic, glass, metal or ceramic or consists of plastic, glass, metal or ceramic.

11. Appliance (10) for inspecting a sample (12) according to claim 10, wherein the appliance (10) is further designed to detect a sample outline (32) and to locate the contamination (30) relative to the sample outline (32).

12. Appliance (10) for inspecting a sample (12) according to claim 10 or 11, wherein the appliance (10) is further designed to reject a quality of the sample (12) if a decay time of the autofluorescence has lower values than expected.

13. Appliance (10) for inspecting a sample (12) according to any one of claims 10 to 12, further comprising: a blade, wherein the appliance (10) is designed to cut a foodstuff.

## Revendications

1. Procédé de vérification d'un échantillon (12) par rapport à une contamination (30), comprenant :

la détermination d'une déviation d'une distribution de durée de déclin (26) d'une autofluorescence de l'échantillon (12) excitée par un rayonnement électromagnétique par rapport à une distribution de durée de déclin (R) attendue ; **caractérisé en ce que**

la contamination (30) comprend du plastique, du verre, du métal ou de la céramique ou est constituée de plastique, de verre, de métal ou de céramique.

2. Procédé de vérification d'un échantillon (12) selon la revendication 1, comprenant en outre : l'indication (36) d'une contamination (30) de l'échantillon (12) lorsque la déviation est associée à la contamination (30).

3. Procédé de vérification d'un échantillon (12) selon la revendication 1 ou 2, comprenant en outre : la décomposition de la distribution de durée de déclin (26) de l'autofluorescence de l'échantillon (12) excitée par le rayonnement électromagnétique en une ou plusieurs parties associées à un matériau de l'échantillon (12) et une ou plusieurs parties résiduelles.

4. Procédé de vérification d'un échantillon (12) selon la revendication 3, comprenant en outre : la mise en correspondance de l'une ou des plusieurs parties associées au matériau de l'échantillon (12) avec une distribution de durée de déclin caractéristique d'un produit alimentaire.

5. Procédé de vérification d'un échantillon (12) selon l'une quelconque des revendications 1 à 4, dans lequel le rayonnement électromagnétique présente une longueur d'onde dans la plage de 425 nm à 550 nm, de préférence 488 nm.

6. Procédé de vérification d'un échantillon (12) selon l'une quelconque des revendications 3 à 5, comprenant en outre :
la mise en correspondance de l'une ou des plusieurs parties résiduelles avec une distribution de durée de déclin de la contamination (30).

7. Procédé de vérification d'un échantillon (12) selon la revendication 1, dans lequel le plastique comprend du polyamide, du polyéthylène, du chlorure de polyvinyle, du polyéthylène téréphtalate, du polystyrène, du polypropylène ou de l'acrylonitrile-butadiène-styrène.

8. Procédé de vérification d'un échantillon (12) selon l'une quelconque des revendications 1 à 7, comprenant en outre :
la comparaison de distributions de durée de déclin (26) de l'autofluorescence excitée par le rayonnement électromagnétique de deux ou plusieurs volumes d'échantillons ou échantillons (12) différents, dans lequel la contamination (30) est indiquée lorsqu'une déviation des deux ou plusieurs distributions de durée de déclin (26) se situe en dehors d'une plage de tolérance.

9. Procédé de vérification d'un échantillon (12) selon la revendication 1, comprenant en outre :

la détermination (40) d'un décalage de la distribution de durée de déclin (26) par rapport à une référence (R) ; et
l'indication (42) d'une détérioration de la qualité lorsque la distribution de durée de déclin (26) se situe en dehors d'une plage de tolérance (T) autour de la référence (R).

10. Appareil (10) de vérification d'un échantillon (12) par rapport à une contamination (30), comprenant :

un émetteur (14) configuré pour émettre un rayonnement électromagnétique modulé en fréquence ou en amplitude ; et
un détecteur (16) configuré pour recevoir une autofluorescence de l'échantillon (12) excitée par le rayonnement électromagnétique ;
dans lequel l'appareil (10) est configuré pour déterminer un résultat de la vérification sur la base d'une distribution de durée de déclin (26) de l'autofluorescence de l'échantillon (12) ;
dans lequel l'appareil (10) est en outre configuré pour indiquer une contamination (30) de l'échantillon (12) lorsque la distribution de temps de déclin (26) de l'autofluorescence de l'échantillon (12) dévie d'une distribution de durée de déclin (R) attendue ;
**caractérisé en ce que**
la contamination (30) comprend du plastique, du verre, du métal ou de la céramique ou est constituée de plastique, de verre, de métal ou de céramique.

11. Appareil (10) de vérification d'un échantillon (12) selon la revendication 10, dans lequel l'appareil (10) est en outre configuré pour détecter un contour d'échantillon (32) et pour localiser la contamination (30) par rapport au contour d'échantillon (32).

12. Appareil (10) de vérification d'un échantillon (12) selon la revendication 10 ou 11, dans lequel l'appareil (10) est en outre configuré pour contester une qualité de l'échantillon (12) lorsqu'une durée de déclin de l'autofluorescence présente des valeurs inférieures à celles attendues.

13. Appareil (10) de vérification d'un échantillon (12) selon l'une quelconque des revendications 10 à 12, comprenant en outre :
un couteau, dans lequel l'appareil (10) est configuré pour découper un aliment.

**Fig. 1**

**Fig. 1a**

**Fig. 1b**

**Fig. 2**

**Fig. 2a**

**Fig. 2b**

**Fig. 3**

**Fig. 3a**

**Fig. 3b**

**Fig. 4**

**Fig. 5**

#Segmente

T

R

26

Abklingzeit

**Fig. 6**

#Segmente

D

26'

28a

28b

Abklingzeit

**Fig. 6a**

**Fig. 6b**

**Fig. 6b'**

**Fig. 6b"**

**Fig. 7**

**Fig. 8a**

**Fig. 8b**

Abklingzeit

$r_2 + T_2/2$

$r_2 - T_2/2$

$r_1 + T_1/2$

$r_1 - T_1/2$

# Probe

**Fig. 9a**

#Segmente

T1

T2

Abklingzeit

**Fig. 9b**

**Fig. 10**

**Fig. 11**

**Fig. 12**

**Fig. 13**

START

ÜBERPRÜFEN, OB EINE ABKLINGZEITVERTEILUNG EINER DURCH
EINE ELEKTROMAGNETISCHE STRAHLUNG ANGEREGTEN
AUTOFLUORESZENZ DER PROBE VON EINER ERWARTETEN
ABKLINGZEITVERTEILUNG ABWEICHT

— 34

ANZEIGEN EINER VERUNREINIGUNG, WENN DAS ÜBERPRÜFEN
ERGIBT, DASS DIE ABKLINGZEITVERTEILUNG DER DURCH DIE
ELEKTROMAGNETISCHE STRAHLUNG ANGEREGTEN
AUTOFLUORESZENZ DER PROBE VON DER ERWARTETEN
ABKLINGZEITVERTEILUNG ABWEICHT

— 36

STOP

**Fig. 14**

START

─ 38

ERFASSEN EINER ABKLINGZEITVERTEILUNG EINER DURCH EINE
ELEKTROMAGNETISCHE STRAHLUNG ANGEREGTEN
AUTOFLUORESZENZ EINER PROBE

─ 40

BESTIMMEN EINER VERSCHIEBUNG DER
ABKLINGZEITVERTEILUNG RELATIV ZU EINER REFERENZ

─ 42

ANZEIGEN EINER QUALITÄTSVERSCHLECHTERUNG, WENN DIE
ABKLINGZEITVERTEILUNG AUSSERHALB EINES
TOLERANZBEREICHS UM DIE REFERENZ LIEGT

STOP

**Fig. 15**

**Fig. 16**

**Fig. 17**

**Fig. 18**

**Fig. 19**

**Fig. 20**

**Fig. 21**

**Fig. 22**

Fig. 23

**Fig. 24**

**Fig. 25**

**Fig. 26**

**Fig. 27**

**Fig. 28**

**Fig. 29**

**Fig. 30**

**Fig. 31**

**Fig. 32**

**Fig. 33**

**Fig. 34**

**Fig. 35**

**Fig. 36**

**Fig. 37**

**Fig. 38**

**Fig. 39**

**Fig. 40**

**Fig. 41**

**Fig. 42**

**Fig. 43**

**Fig. 44**

**Fig. 45**

**Fig. 46**

**Fig. 47**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10315541 A1

- EP 2251675 A1

### In der Beschreibung aufgeführte Nicht-Patentliteratur

- **BOUCHARD ALAIN et al.** Optical characterization of Pseudomonas fluorescens on meat surfaces using time-resolved fluorescence. *JOURNAL OF BIOMED-ICAL OPTICS,* 01. Januar 2006, vol. 11 (1), 014011

- *CHEMICAL ABSTRACTS,* 1260-17-9